# NEUE EUROPÄISCHE PATENTSCHRIFT

(11) **EP 3 000 493 B2**
(45) Veröffentlichungstag und Bekanntmachung des Hinweises auf die Entscheidung über den Einspruch: **02.11.2022**
(45) Hinweis auf die Patenterteilung: 10.05.2017
(21) Anmeldenummer: 15002837.1
(22) Anmeldetag: 27.08.2008
(51) Int. Cl.: A61M 1/10, A61M 1/12

(54) **KATHETER-VORRICHTUNG**
CATHETER DEVICE
DISPOSITIF DE CATHETER

(30) Priorität: 08.10.2007 EP 07019658
(43) Veröffentlichungstag der Anmeldung: 30.03.2016
(62) Teilanmeldung aus: 10008268.4
(73) Patentinhaber: AIS GMBH AACHEN INNOVATIVE SOLUTIONS, 52074 Aachen (DE)
(72) Erfinder: Pfeffer, Joachim Georg, 52070 Aachen (DE); Schmitz-Rode, Thomas, 52070 Aachen (DE); Günther, Rolf W., 52074 Aachen (DE)
(74) Vertreter: Cohausz & Florack

(56) Entgegenhaltungen:
- EP-A1- 2 047 872
- WO-A-2006/020942
- WO-A2-2007/112033
- DE-U1- 29 804 046
- US-A- 5 112 292

## Beschreibung

Die Erfindung betrifft eine Katheter-Vorrichtung, die eine miniaturisierte Pumpe ist.

Für die Behandlung schwer herzkranker Patienten werden zunehmend implantierbare Blutpumpen eingesetzt. Derartige Blutpumpen sind bisher vorwiegend für den langfristigen Einsatz vorgesehen. Es werden aber auch Blutpumpen entwickelt, die für die kurzfristige Herzunterstützung ausgelegt sind und minimal-invasiv eingesetzt werden können. Medizinische Ziele sind dabei die Entlastung und Gesundung des Herzens oder aber die Überbrückung bis zu einer möglichen Herztransplantation. Die Breite des Einsatzgebietes solcher Pumpen hängt einerseits von der Einfachheit der Einbringung in den Körper, andererseits von den realisierbaren technischen Eigenschaften und insbesondere der zuverlässig realisierbaren Betriebsdauer der verfügbaren Pumpensysteme ab. Idealerweise sollte eine solche Blutpumpe für die Kurzfristbehandlung perkutan-intravasal ohne jeglichen chirurgischen Eingriff einsetzbar sein.

Im kardiogenen Schock ist die Auswurfleistung des linken Ventrikels erheblich reduziert. Die verminderte Koronarversorgung kann zum irreversiblen Herzversagen führen. Durch den Einsatz eines temporären linksventrikulären Unterstützungssystems soll die Pumpfunktion des linken Ventrikels teilweise bzw. weitgehend übernommen und die Koronarversorgung verbessert werden. Bei Herzoperationen kann ein solches System links- und rechtsventrikulär eingesetzt werden und eine Herz-Lungenmaschine ersetzen.

Ein perkutan-intravsal implantierbares System, das bisher klinische Bedeutung erlangt hat, ist die intraaortale Ballonpumpe (IABP). Die Intraaortale Ballonpumpe oder intraaortale Gegenpulsation ist ein mechanisches System, das auch zur Unterstützung der Pumpleistung des Herzens bei Patienten mit einem kardiogenen Schock eingesetzt wird. Dabei wird ein Katheter mit einem zylindrisch geformten Kunststoffballon über die Leiste in die Brustschlagader (Aorta thoracalis) vorgeschoben, so dass der Ballon unterhalb des Abgangs der linken Schlüsselbeinarterie (Arteria subclavia sinistra) liegt. Dort wird der Ballon mit einer externen Pumpe rhythmisch mit jeder Herzaktion in der Diastole mit 30-40 cm³ Helium aufgeblasen und in der Systole wieder abgelassen. Auf diese Weise verbessert die Ballonpumpe die Durchblutung des Herzmuskels und auch die aller anderen Organe. Die erzielbare hämodynamische Verbesserung ist jedoch nur sehr begrenzt, da aufgrund des Konstruktionsprinzips der IABP keine aktive Blutförderung stattfindet. Durch eine Gegenpulsation wird lediglich im Rhythmus des Herzschlags die Aorta unterhalb des linken Ventrikels verschlossen und somit das vom Herzen noch ausgeworfene Blut zurückgedrückt und umverteilt, damit auch in die Koronarien. Eine Steigerung des Blutflusses erfolgt nicht.
Eine bekannte transfemoral implantierbare Mikro-Axialpumpe "Hemopump^{™}" der Firma Medtronic Inc, USA, stellt sich nach experimenteller und vorläufiger klinischer Prüfung als erfolgsversprechendes Konzept dar, welches eine ausreichende Linksherzentlastung bewirken kann. Der Ansaugstutzen der Pumpe wird retrograd über die Aortenklappe im linken Ventrikel platziert. Der Pumpenrotor befindet sich am Ende einer Kanüle in der oberen Aorta descendens und wird durch einen externen Motor angetrieben. Nachteil des Systems ist, dass die transfemorale Implantation aufgrund des großen Durchmessers des Rotors nur operativ über eine femorale Arterietomie und gegebenenfalls durch eine Graftankopplung möglich ist. Eine Katheter-Vorrichtung gemäß dem Oberbegriff des Anspruch 1 ist auch aus dem Dokument WO2006020942 A1 bekannt. Aus der WO 99/44651 geht eine durch das Blutgefäßsystem eines Patienten einführbare Axialpumpe hervor. Die Axialpumpe weist ein flexibles komprimierbares Rohr auf, welches das Pumpengehäuse bildet. In dem Rohr befindet sich ein radial komprimierbarer Rotor. Die Antriebswelle des Rotors verläuft durch einen Katheter hindurch. Der Katheter kann zusammen mit dem Rohr und dem Rotor in einen Deckschlauch hineingezogen werden. Die radiale Komprimierbarkeit der Komponenten erlaubt die Realisierung eines für eine perkutane Implantation in Seldinger-Technik vertretbar kleinen Punktionsdurchmessers. Durch die Entfaltung im Herz-Gefäßsystem kann ein relativ großer Pumpendurchmesser von 10 bis 14 mm vorgesehen werden. Hierdurch sinkt die Rotordrehzahl und damit die mechanische Beanspruchung der Komponenten.

In der US 4,753,221 ist ein Katheter mit integrierter Blutpumpe, die klappbare Flügel aufweist, beschrieben. Die Blutpumpe ist eine Axialpumpe, die innerhalb eines Katheterrohres angeordnet ist, an dessen Ende ein Ballon vorgesehen ist, der aufgepumpt werden kann, um den Pumpenmantel zu entfalten und um den an der Pumpe vorbeiführenden Strömungsweg zu verschließen und auf diese Weise die Pumpe im Blutgefäß zu befestigen. In einem weiteren Ausführungsbeispiel ist vorgesehen, ein becherartig ausgebildetes Ende des Katheters in einem röhrenförmigen Führungskatheter anzuordnen, diesen zurückzuziehen und auf diese Weise das becherartige Ende zu entfalten.

Aus der DE 10 059 714 C1 geht eine intravasale Pumpe hervor. Die Pumpe weist ein Antriebsteil und ein Pumpenteil auf, die einen so geringen Durchmesser haben, dass sie durch ein Blutgefäß geschoben werden können. An den Pumpenteil schließt sich eine flexible Kanüle an. Zur Verringerung des Strömungswiderstandes kann die Kanüle auf einen Durchmesser aufgeweitet werden, der größer ist als derjenige des Antriebsteils bzw. des Pumpenteils. Um die Pumpe in Seldinger-Technik durch Punktionen des Blutgefäßes in den Körper einführen zu können, wird die Kanüle in den eingeschnürten Zustand versetzt, in dem sie einen kleinen Durchmesser hat. Im Blutgefäß wird sie aufgeweitet, so dass sie dort einen geringeren Strömungswiderstand für das zu pumpende Blut bietet.

In der JP 4126158 bzw. der EP 0 445 782 A1 ist ein künstliches in den Körper implantierbares Herz beschrieben. Das künstliche Herz weist einen Pumpenabschnitt und einen Antriebsabschnitt zum Antrieb des Pumpenabschnittes auf. Der Pumpenabschnitt ist relativ klein ausgebildet und dient zur Aufnahme einer Axialstrompumpe. Die Axialstrompumpe ist als Schraubenspindelpumpe ausgebildet. Es sind verschiedene Ausführungsformen von Schraubenspindelpumpen vorgesehen.

In der EP 0 364 293 A2 ist ein Katheter mit integrierter Blutpumpe beschrieben. Ein flexibler Rand erstreckt sich über einen rohrförmigen Abschnitt des Katheters und kontaktiert die Wände der Aorta und stellt auf diese Weise sicher, dass das gesamte Blut innerhalb der Aorta durch die Pumpe fließt. Zudem beabstandet der flexible expandierbare Rand die Pumpevon der Aortenklappe.

Aus der US 5,376,114 geht eine Kanülenpumpe hervor, die mittels eines kleinen Einschnittes im Herz temporär eingesetzt werden kann. Die Pumpe weist ein Flügelrad auf, das mittels einer Welle angetrieben wird. Die Welle ist fest mit einem Motormagneten verbunden. Der Motormagnet ist von Magnetspulen umgeben, mit welchen ein sich drehendes Magnetfeld erzeugt werden kann, so dass der Motor in Drehbewegung versetzt wird.

Der vorliegenden Erfindung liegt die Aufgabe zugrunde, eine perkutan-intravasal durch die Femoralarterie einsetzbare Blutpumpe zur Herzunterstützung bereitzustellen, die ohne chirurgischen Eingriff eingeführt werden kann.

Die Aufgabe wird mit einer Katheter-Vorrichtung gemäß Anspruch 1 gelöst. Vorteilhafte Ausgestaltungen der Erfindung sind in den Unteransprüchen angegeben.

Die Katheter-Vorrichtung umfasst eine Antriebswelle, die mit einem Motor verbunden ist und einen Rotor, der am distalen Endbereich auf der Antriebswelle befestigt ist. Der Rotor weist eine Rahmenstruktur auf, die aus einem schraubenförmigen Begrenzungsrahmen und sich radial nach innen vom Begrenzungsrahmen erstreckenden Rotorstreben ausgebildet ist. Die Rotorstreben sind mit ihren vom Begrenzungsrahmen entfernten Enden an der Antriebswelle befestigt. Zwischen den Begrenzungsrahmen und der Antriebswelle erstreckt sich eine elastische Bespannung. Die Rahmenstruktur ist aus einem elastischen Material derart ausgebildet, dass sich der Rotor nach einer aufgezwungenen Kompression selbständig entfaltet.

Durch die Rahmenstruktur des Rotors mit Begrenzungsrahmen und Rotorstreben ist der Rotor sehr stabil, aber dennoch faltbar und lässt sich auf nahezu beliebig kleine Durchmesser komprimieren. Dadurch, dass prinzipiell eine nahezu beliebig lange Ausbildung des Rotors in Längsrichtung und Radialrichtung möglich ist, kann der Rotor je nach dem zur Verfügung stehenden Raum im Hinblick auf eine maximale Förderleistung optimiert werden. Es ist somit möglich, die Förderleistung für eine jede Anwendung optimal anzupassen.

Der Rotor ist derart komprimierbar, dass die Einbringung in den Körper mit einer Punktionsnadel über eine Punktion mit einem Durchmesser von in etwa 9 French (ca. 3 mm) erfolgen kann. Durch das selbständige Entfalten des Rotors wird ein Rotordurchmesser erzielt, der um ein Vielfaches größer als der Durchmesser des Rotors im komprimierten Zustand ist. Hierdurch wird eine hohe Förderleistung erzielt.

Durch den gerüstartigen Aufbau aus Begrenzungsrahmen und Rotorstreben wird dem Rotor eine hohe Festigkeit verliehen, die es erlaubt, den Rotor mit hohen Drehzahlen zu drehen, ohne dass er unwucht wird. Ein Prototyp dieser Katheter-vVorrichtung konnte über mehrere Stunden zum Fördern einer Flüssigkeit mit einer Drehzahl von etwa 32.000 U/min betrieben werden. Der Rotor wies einen Durchmesser von etwa 18 French (=ca. 6 mm) auf und war derart ausgestaltet, dass eine Druckdifferenz von etwa 120 mmHg erzielt wurde. Dies ist für eine derart miniaturisierte Pumpe eine außergewöhnliche Leistung. Mit dieser Katheter-Vorrichtung wurde auch ein deutlicher Fortschritt in Bezug auf Zuverlässigkeit und Lebensdauer erzielt.

Vorzugsweise ist die Rahmenstruktur des Rotors aus einem Formgedächtnismaterial, wie z.B. Nitinol, ausgebildet. Beim Komprimieren kann der Rotor auf eine Temperatur gebracht werden, bei der das Formgedächtnismaterial weich wird. Ein aus Nitinol bestehender Rotor wird beispielsweise bei einer Temperatur von etwa 0°C komprimiert. Beim Erwärmen wird das Formgedächtnismaterial wieder fest und entfaltet sich. In der Regel ist es nicht möglich, den Rotor ohne Abzukühlen wieder zerstörungsfrei zu komprimieren.

Die elastische Bespannung zwischen Begrenzungsrahmen und Antriebswelle ist vorzugsweise aus einer Polymerbeschichtung, wie z.B. PU, PE, PP, Silikon oder Parylene ausgebildet.

Zweckmäßigerweise ist der Rotor von einem rohrförmigen Pumpenabschnitt eines Pumpengehäuses umgeben. Das Pumpengehäuse ist aus einem Gitter ausgebildet, dessen Öffnungen zumindest im Bereich des Pumpenabschnitts mittels einer elastischen Bespannung geschlossen sind. Ein solches Pumpengehäuse kann mit einem geringen Spaltabstand zum Rotor ausgebildet sein, wodurch sich optimale Strömungsbedingungen einstellen und die Förderleistung weiter optimiert werden kann.

Das Gitter des Pumpengehäuses ist vorzugsweise aus einem Formgedächtnismaterial ausgebildet, das zusammen mit dem Rotor komprimierbar ist.

Durch das Pumpengehäuse wird der Rotor von äußeren Einflüssen geschützt.

Die Erfindung wird im folgenden anhand der Zeichnungen beispielhaft näher erläutert. Diese zeigen schematisch in:
- Fig. 1: ein perspektivische Darstellung einer erfindungsgemäßen Katheter-Vorrichtung,
- Fig. 2: eine Explosionszeichnungen einer erfindungsgemäßen Katheter-Vorrichtung ,
- Fig. 3: eine Schaftkappe der Katheter-Vorrichtung in einer seitlich geschnittenen Ansicht,
- Fig. 4: ein distales Katheterschaftstück der Katheter-Vorrichtung in einer seitlich geschnittenen Ansicht,
- Fig. 5: eine Verbindungsbuchse der Katheter-Vorrichtung in einer seitlich geschnittenen Ansicht,
- Fig. 6: eine Pumpe der Katheter-Vorrichtung mit Lagerung in einer seitlich geschnittenen Ansicht,
- Fig. 7a: einen Schnitt entlang der Linie A-A durch die distale Verbindungsbuchse der Katheter-Vorrichtung,
- Fig. 7b: einen Schnitt entlang der Linie B-B durch die proximale Verbindungsbuchse der Katheter-Vorrichtung,
- Fig. 8: eine Gitterstruktur eines Pumpengehäuses der Katheter-Vorrichtung,
- Fig. 9: einen Ausschnitt der Gitterstruktur des Pumpengehäuses der Katheter-Vorrichtung,
- Fig. 10: eine Antriebswelle mit Führungsspirale und Wellenschutz der Katheter-Vorrichtung,
- Fig. 11a: eine Rahmenstruktur eines Rotors einer Pumpe der Katheter-Vorrichtung,
- Fig. 11b: eine weitere Rahmenstruktur des Rotors der Pumpe der Katheter-Vorrichtung,
- Fig. 12: den erfindungsgemäßen Rotor der Pumpe der Katheter-Vorrichtung in einer perspektivischen Ansicht,
- Fig. 13: einen Abströmschlauch der Katheter-Vorrichtung in einer perspektivischen Ansicht,
- Fig. 14: eine erfindungsgemäße Kupplung mit Kupplungsgehäuse und Motor der Katheter-Vorrichtung in einer perspektivischen Ansicht,
- Fig. 15: die erfindungsgemäße Kupplung mit dem Kupplungsgehäuse der Katheter-Vorrichtung in einer perspektivischen Ansicht,

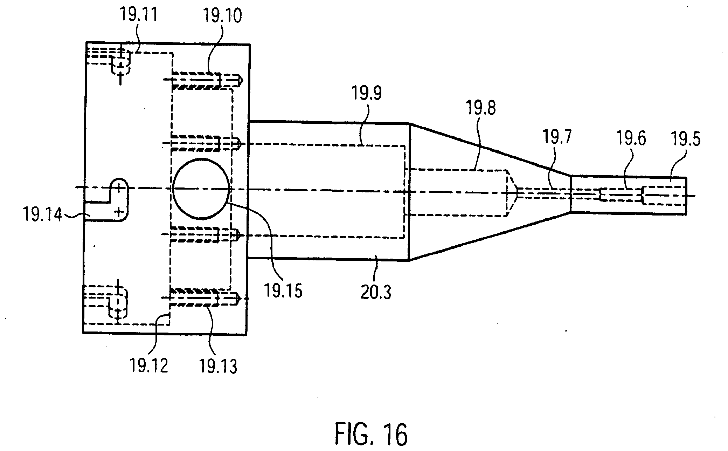

- Fig. 16: das Kupplungsgehäuse der Katheter-Vorrichtung in einer perspektivischen Ansicht,
- Fig. 17: eine Vierkantstange der Kupplung der Katheter-Vorrichtung in einer seitlichen Ansicht,
- Fig. 18: ein Kupplungselement der Kupplung der Katheter-Vorrichtung in einer seitlichen Ansicht,
- Fig. 19: eine Abschlussscheibe der Kupplung der Katheter-Vorrichtung in einer seitlichen Ansicht,
- Fig. 20: eine Kugelkopflagerkugel der Kupplung der Katheter-Vorrichtung in einer seitlichen Ansicht,
- Fig. 21: einen Zentrierstift der Kupplung der Katheter-Vorrichtung in einer seitlichen Ansicht,
- Fig. 22: eine Motoraufnahme der Katheter-Vorrichtung in einer seitlichen Ansicht,
- Fig. 23: das Kupplungselement mit der darin angeordneten Vierkantstange in einer Draufsicht,
- Fig. 24: die im Körper positionierte Katheter-Vorrichtung, und
- Fig. 25: schematisch alternative Ausführungsformen der Katheter-Vorrichtung.

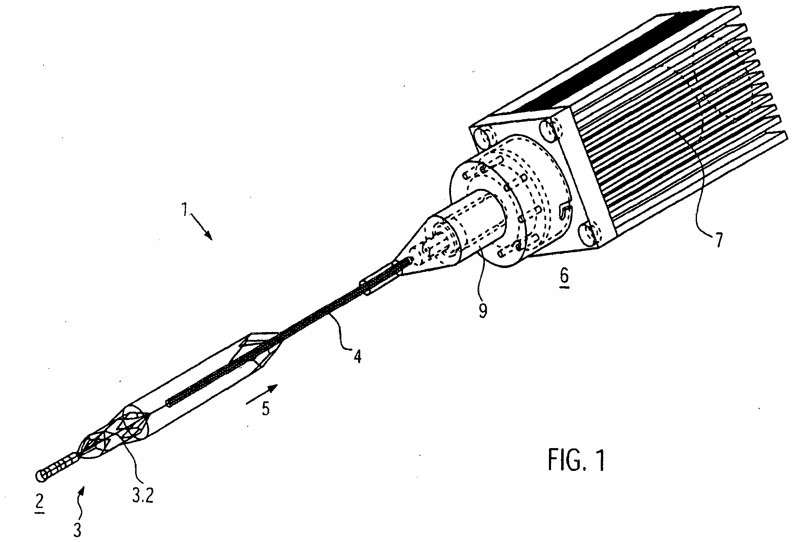

Figur 1 zeigt eine Katheter-Vorrichtung 1. Die erfindungsgemäße Katheter-Vorrichtung 1 stellt eine Pumpe dar. Die Katheter-Vorrichtung 1 weist an einem distalen Ende 2 einen Pumpenkopf 3 auf.

Der Pumpenkopf 3 weist einen Rotor 3.2 zum Fördern eines Mediums in Förderrichtung 5 auf, der mit einer Antriebswelle 4 verbunden ist. Die Förderrichtung 5 ist vom distalen Ende 2 zu einem proximalen Ende 6 gerichtet. An dem vom Pumpenkopf 3 beabstandeten proximalen Ende 6 ist ein Motor 7 angeordnet. Die Antriebswelle 4 ist von einem Katheterschaft 8 umgeben und mittels einer Kupplung 9 kraftschlüssig mit dem Motor 7 verbunden.

Nachfolgend wird zunächst der Pumpenkopf 3 näher erläutert. Der Pumpenkopf 3 umfasst eine Schaftkappe 10 am distalen Ende, den auf der Antriebswelle 4 angeordneten Rotor 3.2, ein Pumpengehäuse 3.1 und einen Abströmschlauch 18.

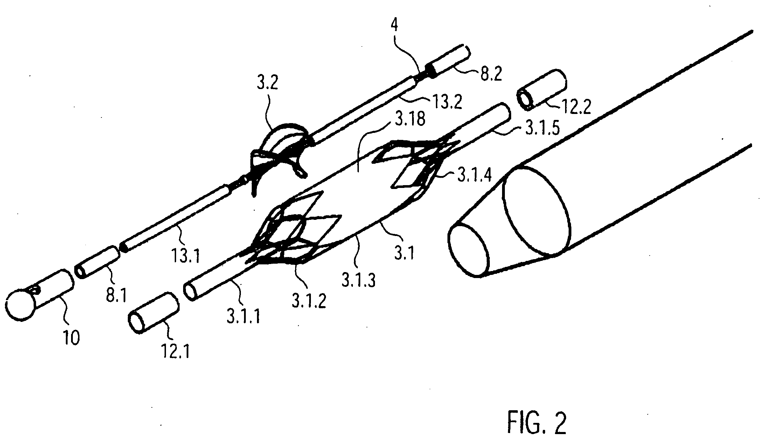

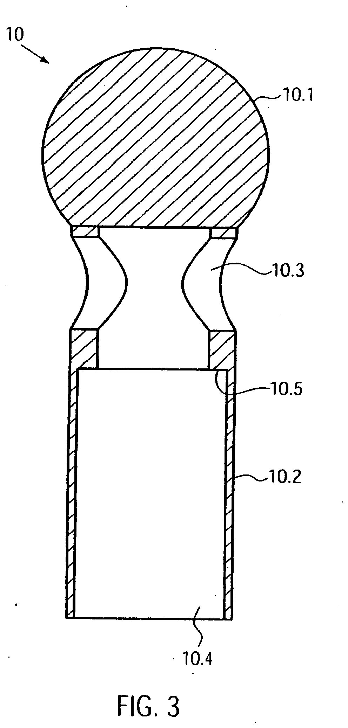

Die Schaftkappe 10 ist aus einer Kugel 10.1 mit einem angesetzten zylinderförmigen Abschnitt 10.2 ausgebildet. Die Schaftkappe 10 ist beispielsweise aus Edelstahl ausgebildet (Fig.2, Fig.3). Die Schaftkappe 10 könnte auch aus Polyethylen PE, Polypropylen PP, Polyetheretherketon PEEK, Polyvinylchlorid PVC, Teflon PTFE, Acrylglas, Epoxydharz, Polyurethan PU, Carbonfiber, beschichteten Materialien, Composite-Materialien, PEBAX, einem Polyether-Block-Amid ausgebildet sein. Prinzipiell sind alle hämokompatiblen Materialien geeignet, da nur eine geringe mechanische Belastung an diesem Bauteil auftritt.

Der Durchmesser der Kugel 10.1 beträgt in etwa 3.2 mm. Der zylinderförmige Abschnitt 10.2 ist in etwa 5,5 mm lang und hat einen Durchmesser von in etwa 2,2 mm. Die Gesamtlänge der Schaftkappe beträgt in etwa 7,0 mm.

Der zylinderförmige Abschnitt 10.2 weist an seinem distalen Ende, im Anschlussbereich zur Kugel 10.1 eine quer zur Förderrichtung 5 angeordnete Durchgangsbohrung 10.3 auf. Weiterhin weist der Zylinder 10.2 eine axiale Bohrung 10.4 auf, die sich vom proximalen Ende des zylinderförmigen Abschnittes 10.2 bis zur Kugel 10.1 hin erstreckt, so dass ein kommunizierender Durchgang von der Durchgangsbohrung 10.3 bis zum proximalen Ende der Schaftkappe 10 ausgebildet ist. Im Bereich der axialen Bohrung 10.4 ist eine Stufe 10.5 ausgebildet, so dass die axiale Bohrung in Richtung des proximalen Endes aufgeweitet ist.

Durch die Durchgangsbohrung 10.3 wird einerseits vermieden, dass in der Schaftkappe ein Sackloch entsteht und andererseits erlaubt das Durchgangsloch die Anbringung eines Fadens, der beim Komprimieren des Pumpenkopfes 3 hilfreich ist.

Anstelle der Kugel 10.1 der Schaftkappe 10 kann auch ein Pigtail, eine Spirale, ein mäanderförmiger Draht mit Kugelspitze oder ein atraumatisches Faserbündel vorgesehen sein. Die Schaftkappe ist aufgrund ihrer geringen Größe bevorzugt.

Die Spitze der Schaftkappe 10 ist eine atraumatische Kugel zum Schutz des Herzmuskels (Endocard). Über die Schaftkappe 10 kann der Pumpenkopf 3 an der Herzwand abgestützt werden.

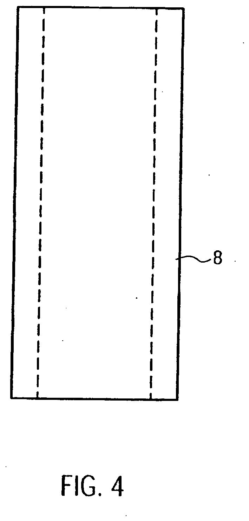

Ein rohrförmiges bzw. schlauchförmiges, distales Katheterschaftstück 8.1 ist vom proximalen Ende in die Schaftkappe 10 bis zur Stufe eingeführt. Das distale Katheterschaftstück 8.1 wird in der axialen Bohrung 10.4 passgenau aufgenommen und ist dort fixiert (Fig. 4). Das distale Katheterschaftstück 8.1 ist aus Polyurethan bzw. aus einem anderen geeigneten Material, insbesondere einem elastischen Kunststoffmaterial (z.B. PE, PVC, Teflon, Elastomer), ausgebildet. Das distale Ende des distalen Katheterschaftstücks 8.1 wird mit der Schaftkappe 10 verbunden. Die Verbindung kann als Klebeverbindung mittels beispielsweise Cyanacrylatkleber ausgebildet sein oder sie erfolgt als Schweiß-, Klemm- oder Schrumpfverbindung. Diese Verbindungsmittel sind grundsätzlich geeignet, ein Katheterschaftstück mit einem anderen, insbesondere steifen Teil zu verbinden. In der nachfolgenden Beschreibung wird dies deshalb nicht an jeder einzelnen Verbindungsstelle ausgeführt.

Das distale Katheterschaftstück 8.1 bildet eine gerade, aber leicht flexible Verbindung zwischen der Schaftkappe 10 und dem Pumpengehäuse 3.1. Die gerade Verbindung stellt eine Koaxialität aller in ihr angeordneten Bauteile (Antriebswelle, Wellenschutz, Gehäuse, Verbindungsbuchse) her.

Das distale Katheterschaftstück 8.1 dient in Verbindung mit der Schaftkappe 10 als Positionierungshilfe des Pumpenkopfes 3 beim Einbringen in ein Gefäß bzw. das Herz.

Das Katheterschaftstück 8.1 im vorliegenden Ausführungsbeispiel weist eine Länge von in etwa 25 mm, einen Außendurchmesser von in etwa 1,9 mm und einen Innendurchmesser von in etwa 1,3 mm auf.

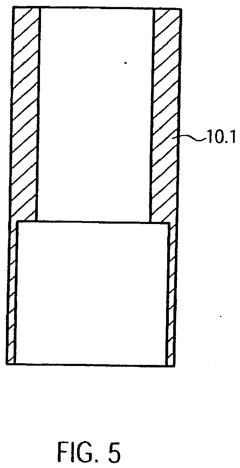

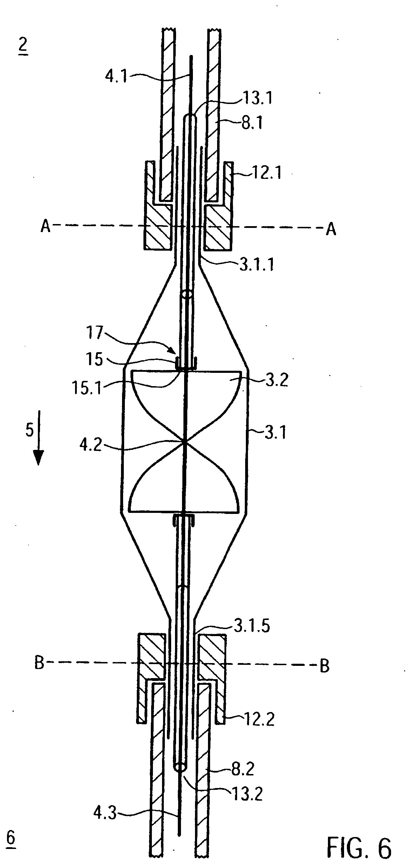

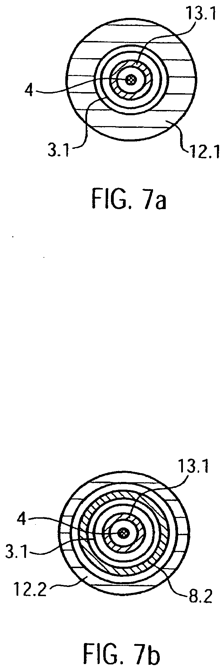

Am proximalen Ende des distalen Katheterschaftstücks 8.1 ist eine distale, röhrenförmige Verbindungsbuchse 12.1 vorgesehen (Fig. 5, Fig. 6). Die distale Verbindungsbuchse 12.1 weist im distalen Bereich einen größeren Innendurchmesser als im proximalen Bereich auf. Im distalen Bereich der Verbindungsbuchse 12.1 ist das proximale Ende des distalen Katheterschaftstücks 8.1 passgenau aufgenommen und fixiert. Im proximalen Bereich der distalen Verbindungsbuchse 12.1 ist ein distaler Verbindungsabschnitt 3.1.1 des Pumpengehäuses 3.1 aufgenommen. Der distale Verbindungsabschnitt 3.1.1 des Pumpengehäuses 3.1 ist mit der distalen Verbindungsbuchse 12.1 und dem proximalen Ende des distalen Katheterschaftstücks 8.1 verbunden (Fig. 7a, Fig. 7b).

Die distale Verbindungsbuchse 12.1 weist eine Länge von in etwa 5 mm und einen Außendurchmesser von in etwa 2,2 mm auf. Im distalen Bereich beträgt der Durchmesser in etwa 2 mm und im proximalen Bereich in etwa 1,5 mm. Je kürzer die Verbindungsbuchse ist, desto geringer ist die hierdurch bewirkte Aussteifung.

Die distale und eine analog ausgebildete proximale Verbindungsbuchse 12.1, 12.2 sind beispielsweise aus Edelstahl, Kupfer, Messing, Titan oder einem anderen geeigneten Metall, aus Polyethylen (PE), Polypropylen (PP), Teflon (PTFE), PEBAX, einem Polyether-Block-Amid, oder einem anderen geeigneten Material ausgebildet.

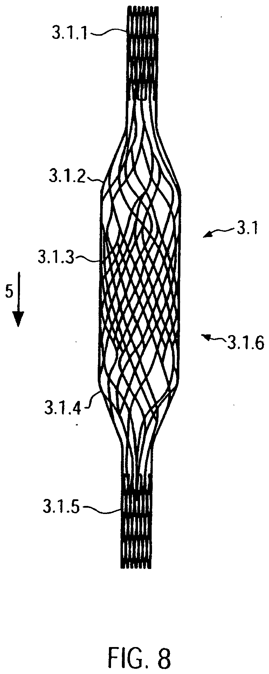

Das expandierbare bzw. komprimierbare Pumpengehäuse 3.1 ist eine rohrförmig ausgebildete Gitterstruktur 3.1.6 aus Nitinol oder einer anderen geeigneten Gedächtnislegierung oder einem anderen Formgedächtnismaterial, z.B. Kunststoff, Eisenlegierung, Kupferlegierung. Das Pumpengehäuse 3.1 ist von distal nach proximal in fünf Abschnitte unterteilt (Fig. 8). Der erste distale Abschnitt ist ein rohrförmig ausgebildeter distaler Verbindungsabschnitt 3.1.1. Ein zweiter Abschnitt ist ein in Förderrichtung 5 konusförmig aufgeweiteter Ansaugabschnitt 3.1.2. An den Ansaugabschnitt 3.1.2 schließt sich ein Pumpenabschnitt 3.1.3 an. Der rohrförmige Pumpenabschnitt 3.1.3 nimmt den Rotor 3.2 auf. Der Innendurchmesser des Pumpenabschnitts 3.1.3 beträgt im expandierten Zustand in etwa 6,15 mm. Ein Auslassabschnitt 3.1.4 verengt sich konusförmig in Förderrichtung 5 und bildet die Verbindung zwischen dem Pumpenabschnitt 3.1.3 und einem proximalen Verbindungsabschnitt 3.1.5. Der proximale Verbindungsabschnitt 3.1.5 ist analog zum distalen Verbindungsabschnitt 3.1.1 rohrförmig mit geringerem Durchmesser als der Pumpenabschnitt 3.1.3 ausgebildet. Das Pumpengehäuse 3.1 kann derart komprimiert werden, dass es über die gesamte Länge einen maximalen Durchmesser von weniger als 3 mm nicht überschreitet.

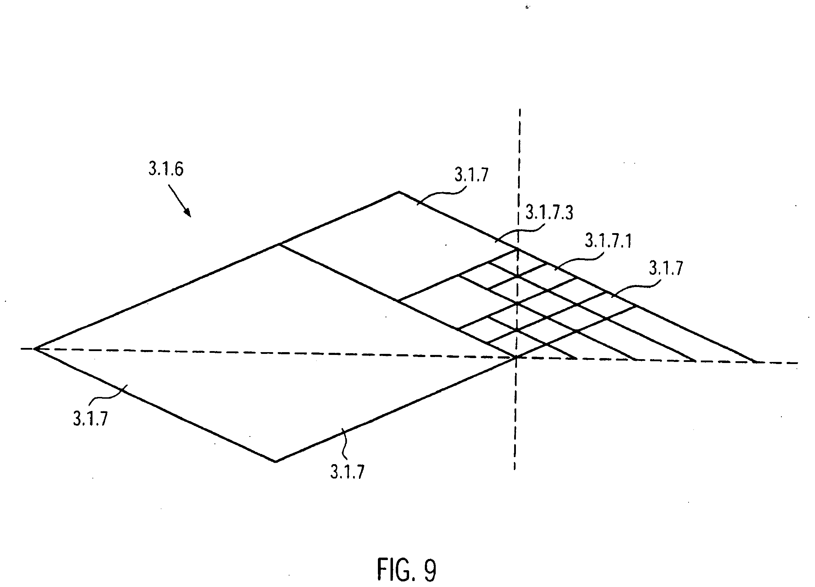

Die Gitterstruktur 3.1.6 des Pumpengehäuses 3.1 weist zwischen den Gitterstreben Öffnungen 3.1.7 auf (Fig. 8, Fig. 9). Die Öffnungen sind als Polygone 3.1.7 ausgebildet, die im vorliegenden Ausführungsbeispiel Rauten sind. Im Pumpenabschnitt 3.1.3 sind kleine Rauten 3.1.7.1 vorgesehen. In den Übergangsbereichen vom Pumpenabschnitt 3.1.3 zum Ansaugabschnitt 3.1.2 und zum Auslassabschnitt 3.1.4 der rohrförmigen Gitterstruktur 3.1.6 sind die kleinen Rauten 3.1.7.1 schrittweise zu größer werdenden Rauten zusammengefasst. Benachbart zu einer kleinen Raute ist eine größere Raute mit doppelter Kantenlänge angeordnet. Diese Verdoppelung der Kantenlänge wird so oft wiederholt, bis die Öffnungen die gewünschte Größe aufweisen. Im Ansaugabschnitt 3.1.2 und im Auslassabschnitt 3.1.4 sind große Rauten 3.1.7.2 vorgesehen, die in etwa die vierfache Kantenlänge der kleinen Rauten 3.1.7.1 aufweisen. In den Übergangsbereichen vom Ansaugabschnitt 3.1.2 und vom Auslassabschnitt 3.1.4 zum distalen und zum proximalen Verbindungsabschnitt 3.8.1, 3.1.5 der rohrförmigen Gitterstruktur 3.1.6 sind die großen Rauten 3.1.7.2 zu kleiner werdenden Rauten zusammengefasst. Im distalen und im proximalen Verbindungsabschnitt sind mittelgroße Rauten 3.1.7.3 vorgesehen, die in etwa die zweifache Kantenlänge der kleinen Rauten 3.1.7.1 aufweisen (Fig. 9). Die Ausbildung der Öffnungen 3.1.7 und die Anzahl der Verfielfachungen kann beliebig sein. Bei dem Übergang von kleineren zu größeren Rauten wird die Breite der Gitterstreben vergrößert. Damit wird die Festigkeit der Gitterstreben in etwa gleichgehalten bzw. zu den größeren Rauten hin sogar verstärkt.

Die Gitterstruktur 3.1.6 des Pumpengehäuses 3.1 ist im Pumpenabschnitt 3.1.3 mit einer PU-Bespannung 3.1.8 bespannt, wodurch die Gitteröffnungen flüssigkeitsdicht verschlossen sind.

Diese Bespannung bzw. die Abdichtung der Gitterstruktur 3.1.6 kann auch z.B. durch einen PU-Schlauch der auf der Oberfläche außen oder innen angeordnet ist, ausgebildet sein.

Es kann auch eine andere Bespannung als PU verwendet werden, wie z.B. PE, PP, Silikon oder Parylene, solange sie die mechanischen und geometrischen Anforderungen erfüllt.

Durch die Auswahl einzelner Öffnungen 3.1.71, insbesondere der mittleren und größeren Öffnungen 3.1.7.3, 3.1.7.2, die nicht beschichtet werden, können die Leistungsparameter inkl. Blutschädigung der Pumpe gezielt gesteuert werden.

Durch die polygonale Struktur und die besondere Ausführung der PU-Bespannung ergibt sich eine annähernd runde Querschnittsform für das Pumpengehäuse 3.1. In Verbindung mit dem runden Rotor 3.2 ergeben sich dadurch sehr geringe Spalte zwischen Rotor 3.2 und Pumpengehäuse 3.1. Dies führt zu vergleichsweise niedriger Blutschädigung, niedrigen Leckströmen und einem guten Wirkungsgrad. Durch die Gitterstruktur 3.1.6 ergibt sich eine sehr gute radiale und axiale Stabilität sowie eine sehr gute axiale Komprimierbarkeit und Expandierbarkeit. Durch die spezielle Struktur kann sehr einfach eine Anpassung von Länge und Durchmesser an die Leistungsanforderungen erfolgen.

Der proximale Verbindungsabschnitt 3.1.5 des Pumpengehäuses 3.1 ist in der proximalen Verbindungsbuchse 12.2 aufgenommen und mit dieser verbunden. In der proximalen Verbindungsbuchse 12.2 ist analog zur distalen Verbindungsbuchse 12.1 ein schlauchförmiges proximales Katheterschaftstück 8.2 aufgenommen und mit dieser verbunden (Fig. 7a, Fig. 7b). Es können die gleichen Verbindungsarten, die bereits oben beschrieben sind, vorgesehen sein.

Innerhalb des distalen und des proximalen Katheterschaftstückes 8.1, 8.2 ist in axialer Richtung ein distaler Wellenschutz 13.1 und ein proximaler Wellenschutz 13.2 angeordnet (Fig. 6). Der distale und der proximale Wellenschutz 13.1, 13.2 sind als Schlauch aus PU oder einem anderen der bereits oben aufgeführten Materialien ausgebildet.

Der distale Wellenschutz 13.1 erstreckt sich in Förderichtung 5 von kurz vor der distalen Verbindungsbuchse 12.1 bis zum distalen Ende des Pumpenabschnitts 3.1.3 des Pumpengehäuses 3.1, d.h. bis zum Rotor 3.2. Der proximale Wellenschutz 13.2 erstreckt sich vom proximalen Ende des Rotors 3.2 bis kurz hinter das proximale Ende der proximalen Verbindungsbuchse 12.1.

Der distale und der proximale Wellenschutz 13.1, 13.2 sind in den beiden Bereichen, in denen sie innerhalb der distalen und der proximalen Verbindungsbuchse 12.1, 12.2 bzw. des distalen und des proximalen Katheterschaftstücks 8.1, 8.2 angeordnet sind, mit diesen verbunden.

Die beiden Verbindungsbuchsen 12.1, 12.2 bilden zusammen mit den darin angeordneten Bauteilen (Wellenschutz, Pumpengehäuse, Katheterschaft) einen Lagerbereich für die Antriebswelle 4. Die Verbindungsbuchsen 12.1, 12.2 gewährleisten die Achszentriertheit der Antriebswelle 4 insbesondere im Pumpengehäuse 3.1.

Innerhalb des distalen und des proximalen Wellenschutzes 13.1, 13.2 bzw. des Pumpengehäuses 3.1 ist in axialer Richtung die Antriebswelle 4 angeordnet. Die Antriebswelle 4 weist in Förderichtung 5 drei Abschnitte auf. Einen distalen Abschnitt der Antriebswelle 4.1 im Bereich der Schaftkappe 10. Einen Pumpenabschnitt der Antriebswelle 4.2, auf dem der Rotor 3.2 drehfest angeordnet ist und einen proximalen Abschnitt der Antriebswelle 4.3, der sich vom Pumpenabschnitt 3.1.3 bis hin zur Kupplung 9 erstreckt. Der Rotor 3.2. ist mit der Antriebswelle verklebt. Es können jedoch auch andere kraftschlüssige Verbindungen wie Schweißen oder Klemmen vorgesehen sein.

Der proximale Wellenschutz 13.2 (Fig. 2, Fig. 6) trennt, zum Schutz vor Blutschädigung durch die Rotationsbewegung der Antriebswelle 4 und Anhaftung von Blutbestandteilen an der Antriebswelle 4, den proximalen Abschnitt 4.3 der Antriebswelle 4 räumlich vom Pumpmedium. Dadurch bauen sich keine Scherkräfte auf. Es kommt zu keiner direkten Wechselwirkung zwischen der Antriebswelle 4 und dem Blut durch den sehr geringen Spalt und es ist lediglich ein minimaler Bluttransport durch diesen Spalt möglich. Der distale und der proximale Wellenschutz 13.1, 13.2 zentrieren und stützten die Antriebswelle 4 im Betrieb und während des Komprimierungs- und Expansionsvorgangs.

Die Antriebswelle 4 ist bevorzugt aus mehreren, insbesondere sechs Drähten (nicht dargestellt), die links- oder rechtsgewickelt um eine Seele (nicht dargestellt) angeordnet sind, ausgebildet. Der Außendurchmesser der Antriebswelle 4 beträgt in etwa 0,48 mm. Die Antriebswelle 4 kann aber auch eine andere Anzahl von Seelen und Drähten aufweisen und einen kleineren oder einen größeren Durchmesser aufweisen. Der Durchmesser der Antriebswelle kann im Bereich von 0,3 mm bis 1 mm liegen und beträgt vorzugsweise etwa 0,4 mm bis 0,6 mm. Je kleiner der Durchmesser der Antriebswelle ist, desto größer kann die Drehgeschwindigkeit sein, denn je kleiner der Durchmesser ist, desto geringer ist die Geschwindigkeit, mit der sich der Umfang der Antriebswelle gegenüber seiner Umgebung bewegt. Eine hohe Umfangsgeschwindigkeit ist problematisch, wenn die Antriebswelle mit der Umgebung in Berührung kommt. Die Katheter-Vorrichtung ist für Drehzahlen von mehr als 20.000 U/min und bis zu 40.000 U/min ausgelegt. Daher wird der Durchmesser der Antriebswelle 4 so gering wie möglich ausgebildet, aber so dick, dass sie noch eine ausreichende Festigkeit besitzt.

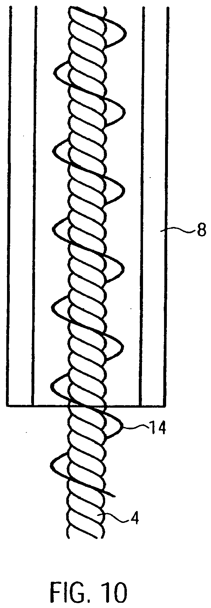

Entgegen der Wickelrichtung der Antriebswelle 4 - im vorliegenden Ausführungsbeispiel ist sie links gewickelt - ist in axialer Richtung um den distalen und den proximalen Abschnitt der Antriebswelle 4.1, 4.3 ein entgegengesetzt gewickelte (hier: rechtsdrehend), spiralförmig ausgebildete Führungsspirale 14 angeordnet, um die Reibung der Antriebswelle 4 zu minimieren, den Wandkontakt der Antriebswelle 4 mit dem proximalen Katheterschaftstück 8.2 zu vermeiden sowie ein Abknicken der Antriebswelle 4 in Folge von Biegung zu verhindern. Durch die Führungsspirale 14 wird die Antriebswelle 4 geführt und geschient bzw. stabilisiert (Fig. 10). Die Führungsspirale 14 kann aus Edelstahl ausgebildet sein und mit dem Wellenschutz 13.1, 13.2 verklebt sein. Es kann auch vorgesehen sein, die Führungsspirale als Feder auszubilden. Die Wickelrichtung der Führungsspirale 14 kann auch gleich der Wickelrichtung der Antriebswelle 4 sein.

Die Antriebswelle 4 erstreckt sich vom distalen Ende des distalen Wellenschutzes 13.1 in Förderrichtung 5 hinter der distalen Verbindungsbuchse 12.1 bis hin zur Kupplung 9.

Das proximale Katheterschaftstück 8.2 stellt in Verbindung mit der Führungsspirale 14 eine längen- und verwindungskonstante Verbindung zwischen dem Pumpenkopf 3 und der Kupplung 9 bereit.

Am proximalen Ende des distalen Wellenschutzes 13.1 ist eine Lagerscheibe 15 angeordnet (Fig. 6). Die Lagerscheibe 15 ist mit einer Durchgangsbohrung 15.1 versehen. Der Durchmesser der Durchgangsbohrung 15.1 entspricht in etwa dem Außendurchmesser der Antriebswelle 4. Die Lagerscheibe 15 wird derart auf der Antriebswelle 4 angeordnet, dass sie das proximale Ende des distalen Wellenschutzes 13.1 aufnimmt und in Förderrichtung 5 begrenzt.

Die Lagerscheibe 15 ist beispielsweise aus Edelstahl, Teflon oder Keramik bzw. einem anderen geeigneten Material ausgebildet. Die Lagerscheibe 15 ist mittels Cyanacrylatkleber mit dem feststehenden Wellenschutz verbunden und kann daher Axialkräfte entgegen der Förderichtung 5 aufnehmen (Verbindungsmittel s.o.).

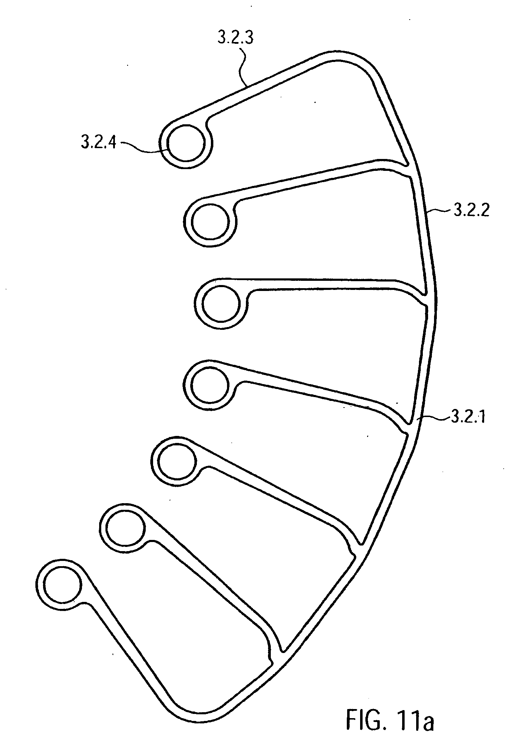

Im Pumpenabschnitt 4.2 der Antriebswelle 4 ist der spiralförmige, expandierbare Rotor 3.2 drehfest auf der Antriebswelle 4 angeordnet. Als Rotor 3.2 ist im vorliegenden Ausführungsbeispiel eine zweiflügelige, kammförmig ausgebildete Rahmenstruktur 3.2.1 aus Nitinol oder einem anderen Formgedächtnismaterial, z.B. Kunststoff (s.o.) vorgesehen, die mit einer PU-Haut beschichtet bzw. von dieser flüssigkeitsdicht umgeben ist (Fig. 11a). D.h. die Bespannung in Form der PU-Haut ist zwischen der kammförmigen Rahmenstruktur aufgespannt. Durch den Aufbau des Rotors 3.2 als beschichtete Rahmenstruktur 3.2.1 aus Nitinol ist es möglich, den Rotor 3.2 zu expandieren bzw. zu komprimieren. Die PU-Haut besitzt eine hohe Elastizität, so dass sie beim Komprimieren nicht beschädigt wird.

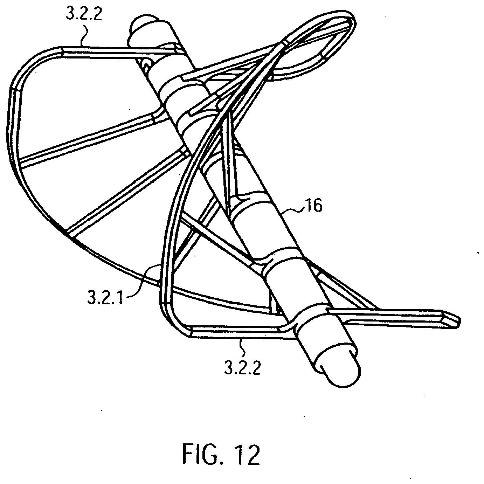

Die Rahmenstruktur 3.2.1 weist einen umlaufenden, schraubenförmigen bzw. spiralförmigen äußeren Begrenzungsrahmen 3.2.2 mit mehreren mit dem Begrenzungsrahmen 3.2.2 verbundenen und radial nach innen verlaufenden Rotorstreben 3.2.3 auf (Fig. 12). An den freien Enden der Rotorstreben 3.2.3 sind Ringe 3.2.4 ausgebildet. Durch die Ringe 3.2.4 der Rotorstreben 3.2.3 erstreckt sich die Antriebswelle 4.

Zwischen zwei benachbarten Ringen 3.2.4 ist jeweils eine Abstandshülse 16 angeordnet. Das distale Ende des Rotors 3.2 liegt mit einer distal endseitigen Abstandshülse 16 an der Lagerscheibe 15 an. Die endseitige Abstandshülse 16 kann auch als spezielle Lagerabstandshülse 16 ausgebildet sein. Auf diese Weise bilden zwei der Rahmenstrukturen 3.2.1 einen zweiflügeligen Rotor 3.2 aus.

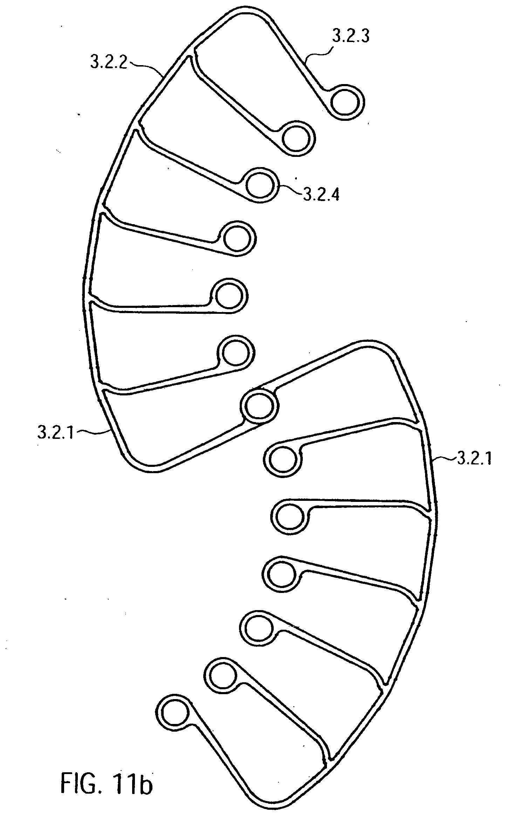

Der Rotor 3.2 kann auch einteilig (Fig. 11b) ausgebildet sein oder mehrere Rahmenstrukturen aufweisen (Fig. 11a). Eine jede Rahmenstruktur bildet einen Rotorflügel. In Fig. 11b und 12 ist eine Rahmenstruktur 3.2.1 für einen Rotor 3.2 dargestellt, die zwei Rotorflügel bildet. Bei Bedarf können auch mehrere Rotorflügel und dementsprechend mehrere Rahmenstrukturen 3.2.1 an einem Rotor 3.2 angeordnet sein. Die Rahmenstruktur kann auch eine beliebige andere geeignete Form aufweisen.

Der Abstand zwischen zwei benachbarten Ringen 3.2.4 ist kleiner als der entsprechende Abschnitt des spiralförmigen Begrenzungsrahmens 3.2.2. Je größer die Differenz zwischen dem Abstand zwischen zwei Ringen 3.2.4 und dem entsprechenden Abschnitt des spiralförmigen Begrenzungsrahmens 3.2.2 ist, desto größer ist die Steigung des Rotors. Durch die Länge der Abstandshülsen 16 kann somit die Steigung des Rotors 3.2 festgelegt werden. Sie kann innerhalb eines Rotors 3.2 variieren.

Durch die Länge bzw. Anzahl der Abstandshülsen 16 im Verhältnis zur Dimensionierung des umlaufenden, spiralförmigen äußeren Begrenzungsrahmens 3.2.2 zwischen zwei Rotorstreben 3.2.3 wird die Steigung des Rotors 3.2 festgelegt. Die Länge der Abstandshülsen 16 kann für alle Positionen einheitlich sein, sie kann aber auch symmetrisch oder asymmetrisch für jede Position variiert werden. Durch die völlige Gestaltungsfreiheit kann ein sehr flexibles Design des Rotors 3.2 erreicht werden. Durch das flexible Design ist es möglich, unterschiedliche Förder- bzw. Pumpeigenschaften des Rotors 3.2 zu generieren.

Der Rotor 3.2 weist eine hohe Formstabilität bei flexibler Gestaltungsmöglichkeit und bei minimalem Materialeinsatz (z.B. dünne Rahmenstruktur) auf. Es wird eine maximale Steifigkeit und Stabilität erreicht. Trotzdem erlaubt die Kombination aus der Rahmenstruktur mit der Bespannung, die die Eigenschaften der Rahmenstruktur durch Stabilisierung weiter unterstützt, eine sehr starke Komprimierung. Dies führt zu der sehr guten Komprimierbarkeit und Expandierbarkeit des Rotors. Aufgrund der guten Flächenausbildung der PU-Haut auf der Gitterstruktur ist eine sehr gute Anpassung der Gehäusestruktur an die Rotorstruktur möglich.

Der Rotor 3.2 weist im komprimierten Zustand in etwa den Innendurchmesser des komprimierten Pumpengehäuses 3.1 auf. Der Außendurchmesser des komprimierten Pumpengehäuses beträgt in etwa zwischen 2 mm bis 4 mm und vorzugsweise in etwa 3,3 mm.

Im expandierten Zustand ist der spiralförmige äußere Begrenzungsrahmen 3.2.2 des Rotors 3.2 geringfügig beabstandet zur Innenfläche des Pumpengehäuses 3.1. Der Abstand zwischen dem äußeren Begrenzungsrahmen 3.2.2 und der Innenfläche des Pumpengehäuses 3.1 beträgt in etwa zwischen 0,01 mm und 0,5 mm. Je kleiner der Abstand zwischen der Rahmenstruktur 3.2.1 und der Innenfläche des Pumpengehäuses 3.1 ist, desto höher ist die Förderleistung des Rotors 3.2.

An der distal endseitigen Abstandshülse 16 des Rotors berühren sich die am distalen Wellenschutz 13.1 befestigte Lagerscheibe 15 und die distal endseitige Abstandshülse 16, die beide auf der Antriebswelle 4 angeordnet sind. Indem der Rotor 3.2 durch die Antriebswelle 4 in eine Drehbewegung versetzt wird, kontaktiert die distale Abstandshülse 16 des Rotors 3.2 die Lagerscheibe 15 in Art eines Gleitlagers. Auf diese Weise ist ein distales Rotorlager 17 ausgebildet (Fig.6). Die Antriebswelle 4 ist von der Durchgangsbohrung der Lagerscheibe 15 nahezu spielfrei aufgenommen. Es verbleiben lediglich kleine Freiräume (nicht dargestellt) aufgrund der Ausbildung der Antriebswelle 4.

Beim Pumpen wird der Rotor 3.2 aufgrund der Förderung des Pumpmediums mit einer axialen Kraft entgegen der Förderrichtung 5 belastet. Diese Kraft wird über die distal endseitige Abstandshülse 16 auf die Lagerscheibe 15 abgeleitet.

Zum Schmieren des distalen Rotorlagers wird über die Durchgangsbohrung 10.3 der Schaftkappe 10, den Freiräumen zwischen dem distalen Wellenschutz 13.1 und der Antriebswelle 4 und dem Freiraum zwischen der Antriebswelle und der Lagerscheibe 15 Blut bzw. Serum eingesogen. Die Sogwirkung entsteht aufgrund der Drehbewegung der Antriebswelle 4 und des Rotors 3.2.

An der proximal endseitigen Abstandshülse 16 des Rotors 3.2 wird die Antriebswelle 4 analog von einer proximalen Verbindungsbuchse 12.2 aufgenommen.

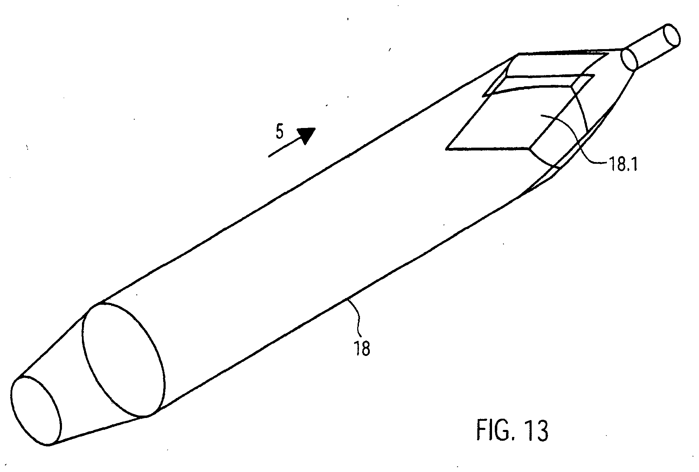

In etwa am proximalen Ende des Pumpenabschnitts 3.1.3 des Pumpengehäuses ist ein rohrförmiger elastischer Abströmschlauch 18 angeordnet (Fig. 1, Fig. 13). Der Abströmschlauch 18 ist aus PU ausgebildet. Der Abströmschlauch 18 weist eine Länge von in etwa 70 mm, einen Durchmesser von in etwa 10 mm und eine Wandstärke von in etwa 0,01 mm bis 0,1 mm und vorzugsweise in etwa 0,03 mm auf. Die beiden Enden des Abströmschlauches 18 sind konisch zulaufend ausgebildet, wobei am proximalen konischen Ende des Abströmschlauches ein zylinderförmiger Abschnitt angeordnet ist.

Das distale konisch zulaufende Ende des Abströmschlauches 18 schließt dicht mit der PU-Bespannung des Pumpenabschnittes 3.1.3 des Pumpengehäuses 3.1 ab. Der zylinderförmige proximale Abschnitt ist fest mit dem proximalen Katheterschaftstück 8.2 verbunden. Beide sind mittels gelöstem PU miteinander flüssigkeitsdicht verbunden.

Am proximalen Ende des Abströmschlauches 18 sind radial umlaufend mehrere Auslassöffnungen 18.1 angeordnet. Die Auslassöffnungen 18.1 können z.B. in Förderrichtung 5 oval ausgebildet sein. Es kann auch vorgesehen sein die Auslassöffnungen rund, halbmondförmig bzw. in einer beliebigen Geometrie auszubilden um andere Auslassströmungen zu generieren. Die Auslassöffnungen 18.1 verwirbeln das in den Bulbus Aorticus austretende Blut. Dadurch wird eine laminare Strömung und damit der Wasserstrahlpumpenefekt gegenüber den Koronararterien verhindert.

Der Abströmschlauch 18 leitet das Pumpvolumen der Pumpe aus dem linken Ventrikel über die Aortenklappe in die Aorta. Hierbei wirkt der Abströmschlauch 18 wie ein Rückschlagventil. Bei positiver Druckdifferenz zwischen Abströmschlauch 18 und Aorta ist der Abströmschlauch 18 entsprechend der von der Pumpe erzeugten Durchflussmenge mehr oder weniger geöffnet. Bei Null oder negativer Druckdifferenz schließt sich der Abströmschlauch 18 aufgrund seiner hohen Flexibilität genau wie die Aortenklappe und legt sich eng an das proximale Katheterschaftstück 8.2 an. Diese Flexibilität führt zu einer guten Abdichtung während der Durchströmung gegenüber den Segeln der Aortenklappe. Auf diese Weise kommt es lediglich zu geringen Rückflüssen aus der Aorta in den linken Ventrikel.

Am proximalen Ende des Katheterschaftes 8.2 sind die Kupplung 9 und der Motor 7 angeordnet. Der Abstand zwischen dem Pumpenkopf 3 und der Kupplung 9 bzw. die Länge des proximalen Katheterschaftstücks 8.2 kann je nach Patient variieren und beträgt in etwa 90 bis 150 cm.

Nachfolgend wird das Verfahren zum Expandieren des Rotors 3.2 beschrieben.

Über der Katheter-Vorrichtung 1 ist ein rohrförmiger Deckschlauch 29 angeordnet. Der Deckschlauch 29 ist derart ausgebildet, dass er den komprimierten Pumpenkopf 3 sowie das proximale Katheterschaftstück 8.2 umgibt. Durch den Deckschlauch 29 wird der Pumpenkopf 3 in seinem komprimierten Zustand gehalten.

Nach der korrekten Positionierung des Pumpenkopfes 3 wird der Deckschlauch 29 von der fixierten Katheter-Vorrichtung 1 zurückgezogen, bis der Pumpenkopf 3 frei liegt. Pumpengehäuse 3.1 und Rotor 3.2 entfalten sich aufgrund der Federkraft des elastischen Materials radial nach außen. Das heißt, die Gitterstruktur 3.1.6 des Pumpengehäuses 3.1 und die Rahmenstruktur 3.2.1 des Rotors 3.2 weiten sich auf bis sie ihren vorgegebenen Durchmesser erreicht haben. Es kann auch vorgesehen sein Temperatureffekte des Memory-Materials beim Expandieren unterstützend zu nutzen.

Zur Entfernung der Katheter-Vorrichtung 1 wird der Deckschlauch 29 bis zur Schaftkappe 10 vorgeschoben, wodurch der Rotor 3.2 und das Pumpengehäuse 3.1 komprimiert werden und in den Deckschlauch eingezogen werden, wonach dieser durch die Punktionsstelle hindurch extrahiert wird.

Nachfolgend werden die Kupplung 9 und der Motor 7 erläutert.

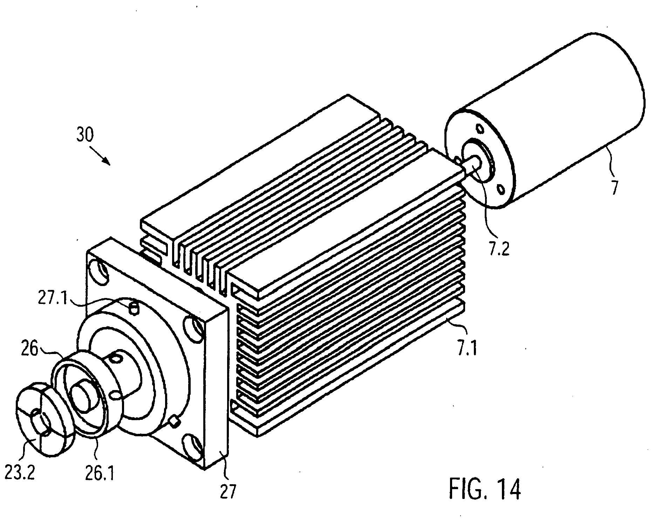

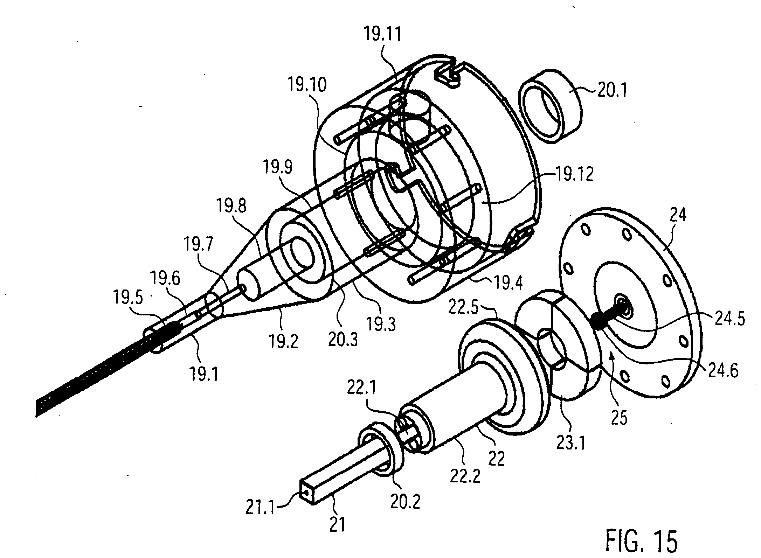

Die Kupplung 9 ist eine Magnetkupplung (Fig. 14, Fig. 15). Die Kupplung 9 weist einKupplungsgehäuse 19 mit einer distalen Magneteinheit 23.1 auf. Das Kupplungsgehäuse 19 ist mit dem proximalen Katheterschaftstück 8.2, das einen durchgehenden Hohlraum ausbildet, verbunden. Das Kupplungsgehäuse 19 trennt das proximale Katheterschaftstück 8.2 hermetisch von einer Motoranordnung 30. Die Motoranordnung 30 weist eine proximale Magneteinheit 23.2 auf. Die proximale Magneteinheit 23.2 ist kraftschlüssig mit dem Motor 7 verbunden. Die distale Magneteinheit 23.1 ist über ein Kupplungselement 22 mit der Antriebswelle 4 verbunden.

Die distale Magneteinheit 23.1 und die proximale Magneteinheit 23.2 sind über Magnetkräfte drehfest miteinander gekoppelt. Durch die beiden Magneteinheiten 23.1, 23.2 wird eine eine kraftschlüssige Verbindung bei berührungfreier rotatorischer Kraftübertragung gewährleistet.

Das Kupplungsgehäuse 19 weist von distal nach proximal einen distalen zylinderförmigen Abschnitt 19.1, einen sich konisch aufweitenden Abschnitt 19.2, einen zweiten zylinderförmigen Abschnitt 19.3 und einen proximalen zylinderförmigen Abschnitt 19.4 auf. Das Kupplungsgehäuse ist z.B. aus Polymethylacrylat (PMMA) ausgebildet oder einem anderen spritzgussfähigen oder zu spanenden Material hergestellt.

Im distalen zylinderförmigen Abschnitt 19.1 ist eine in axialer Richtung mittig angeordnete Durchgangsbohrung ausgebildet. Die Durchgangsbohrung erstreckt sich durch das gesamte Kupplungsgehäuse 19.

Vom distalen Ende des distalen zylinderförmigen Abschnitts 19.1 verengt sich die Durchgangsbohrung dreistufig von einem ersten Katheterschaftaufnahmeabschnitt 19.5 auf einen zweiten Führungsspiraleaufnahmeabschnitt 19.6 und auf einen dritten Antriebswellendurchtrittsabschnitt 19.7.

Der Bohrungsdurchmesser des Katheterschaftaufnahmeabschnitts 19.5 beträgt in etwa 1,9 mm, der des Führungsspiraleaufnahmeabschnitts 19.6 in etwa 1,28 mm und der des dritten Bohrungsabschnitts in etwa 1,0 mm.

Das proximale Ende des proximalen Katheterschaftes ist im Katheterschaftaufnahmeabschnitt 19.5 des Kupplungsgehäuses 19 angeordnet und mit diesem fest verbunden. Im Führungsspiraleaufnahmeabschnitt 19.6 ist die Führungsspirale 14 aufgenommen.

Die Antriebswelle 4 erstreckt sich durch die Durchgangsbohrung des Antriebswellendurchtrittsabschnitts 19.7 des distalen zylinderförmigen Abschnitts 19.1 und des sich konisch aufweitenden Abschnitts 19.1, 19.2. Der Antriebswellendurchtrittsabschnitt 19.7 weitet sich im sich konisch aufweitenden Abschnitt 19.2 in einen vierten Bohrungsabschnitt 19.8 auf.

Der vierte Bohrungsabschnitt geht zu Beginn des zweiten zylinderförmigen Abschnitts 19.3 in einen hohlzylindrischen Lagerungsabschnitt 19.9 über. Im distalen Endbereich des Lagerungsabschnitts 19.9 ist ein äußerer Ringmagnet 20.1 angeordnet. Der äußere Ringmagnet 20.1 ist über eine Presspassung in der Bohrung des Lagerungsabschnitts 19.9 fixiert und kann zusätzlich oder alternativ mittels einer Klebung fixiert sein.

Der Lagerungsabschnitt 19.9 weist einen Durchmesser von in etwa 10 mm auf.

Am Anfang des proximalen zylinderförmigen Abschnitts 19.4 des Kupplungsgehäuses 19 geht die Bohrung des Lagerungsabschnitts 19.9 in einen größeren sechsten distalen Kupplungsabschnitt 19.10 über. Im distalen Kupplungsabschnitt 19.10 ist eine radial angeordnete Spülbohrung 19.15 ausgebildet.

An der Spülbohrung ist eine Pumpe (nicht dargestellt) zum Einbringen eines Mediums, z.B. NaCl, Glukoselösung, Ringerlösung, Plasmaexpander, etc. angeschlossen.

Die Bohrung des distalen Kupplungsabschnitts 19.10 geht in einen größeren proximalen Kupplungsabschnitt 19.11 über. Im zwischen dem distalen und dem proximalen Kupplungsabschnitt 19.10, 19.11 ausgebildeten Absatz 19.12 sind radial symmetrisch 8 x M 1,6 Gewindebohrungen 19.13 ausgebildet. Am proximalen Ende des proximalen Abschnitts 19.4 sind drei L-förmige Ausfräsungen 19.14, auf den Umfang verteilt, angeordnet.

Der distale Kupplungsabschnitt 19.10 weist einen Durchmesser von in etwa 22 mm auf. Die Spülbohrung 19.15 weist einen Durchmesser von in etwa 6,5 mm auf und der proximale Kupplungsabschnitt 19.11 weist einen Durchmesser von in etwa 30 mm auf.

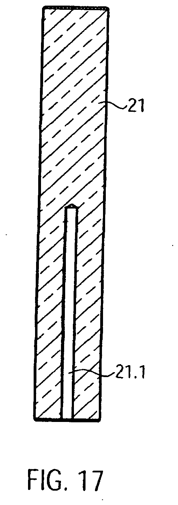

Das proximale Ende der Antriebswelle 4 ist dreh, zug- und druckfest (kraftschlüssig) mit einer quaderförmigen Vierkantstange 21 verbunden (Fig.17). In axialer Richtung weist die Vierkantstange 21 eine Ausnehmung 21.1 zur Aufnahme des proximalen Endes der Antriebswelle 4 auf. Die Antriebswelle 4 ist in der Ausnehmung fixiert. Die Vierkantstange 21 ist z.B. aus Messing, das gute Schmiereigenschaften aufweist, ausgebildet. Weitere geeignete Materialien sind alle zu extrudierenden oder zu spanenden Materialien, wie z.B. PE, PP, PTFE, Gold, Silber, Titan, Diamant, etc..

Die Vierkantstange 21 weist eine Länge von in etwa 19,4 mm und einen Querschnitt von in etwa 2,88 mm x 2,88 mm auf.

Die Vierkantstange 21 überträgt die Drehbewegung des Motors auf die Antriebswelle. Die Vierkantstange 21 kann jede beliebige geometrische Form aufweisen, die einen statisch bestimmten Krafteintrag ermöglicht.

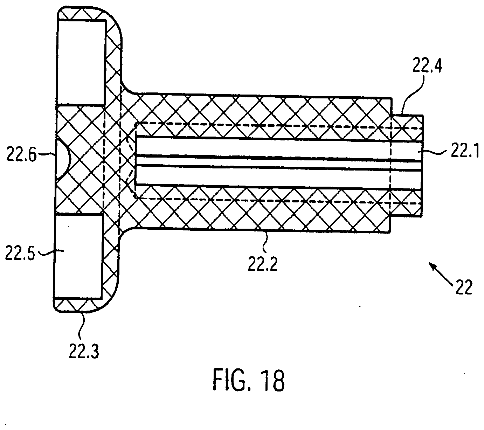

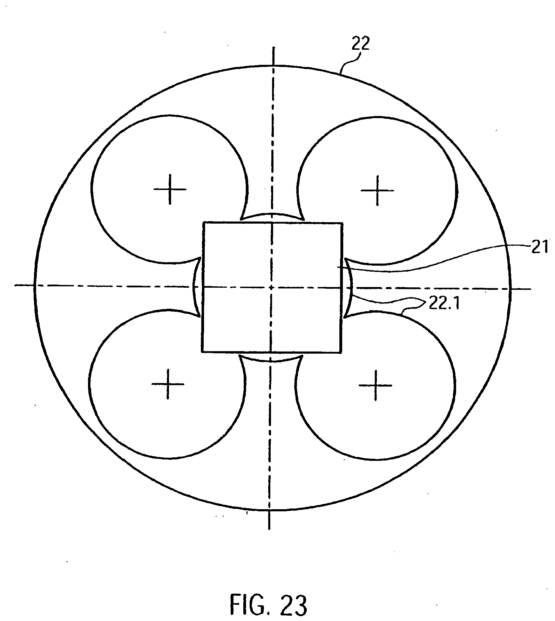

Die Vierkantstange 21 wird von einer axialen Ausnehmung 22.1 innerhalb eines rotationssymmetrischen Kupplungselements 22 axial verschiebbar aufgenommen (Fig. 23). Dadurch ist sie in der Lage Längenunterschiede in axialer Richtung auszugleichen (Fig. 18). Die Ausnehmung 22.1 ist durch eine größere, zentrale Bohrung und vier entlang des Umfangs der zentralen Bohrung angeordnete kleinere Bohrungen ausgebildet. Die Bohrungen können durch Bohren, Erodieren, Ultraschallbohren, Laserbohren oder Wasserstrahlbohren ausgebildet sein.

Durch die Anordnung der Bohrungen werden vier axial verlaufende Doppelanschlagkanten bereitgestellt. Die Ausnehmung 22.1 ist innerhalb eines zylinderförmigen Abschnitts 22.2 des Kupplungselements 22 angeordnet und erstreckt sich vom distalen Ende des Kupplungselements 22 bis kurz vor einen scheibenförmigen proximalen Abschnitt 22.3 des Kupplungselements 22.

Der zylinderförmige Abschnitt 22.2 weist einen Außendurchmesser von in etwa 8 mm auf und der scheibenförmige Abschnitt 22.3 weist einen Außendurchmesser von in etwa 18 mm auf.

Die Ausnehmung 22.1 ist derart ausgebildet, dass die Vierkantstange 21 radial bzw. in Umfangsrichtung fixiert und axial verschieblich aufgenommen wird. Die radiale Fixierung der Vierkantstange 21 erfolgt durch die Kontaktierung aller vier Längskanten der Vierkantstange 21 mit jeweils einer der vier Doppelanschlagkanten der Ausnehmung 22.1. Bei einer axialen Verschiebung der Vierkantstange 21 in der Ausnehmung 22.1 ergibt sich an den entsprechenden Berührungslinien nur eine minimale Reibung.

Es können auch mehr oder weniger Anschlagkanten vorgesehen sein. Anstelle einer Vierkantstange kann z.B auch eine Dreikant- oder Fünfkantstange oder eine Profilstange mit einer an sich beliebigen in Längsrichtung der Stange gleich bleibender Querschnittsfläche vorgesehen sein. Die Ausnehmung 22.1 ist in der Form entsprechend an die Querschnittsfläche der Profilstange anzupassen.

Am distalen äußeren Ende bzw. Umfang des zylinderförmigen Abschnitts 22.2 des Kupplungselements 22 ist ein Absatz 22.4 ausgebildet. Auf diesem Absatz 22.4 ist ein zweiter innerer Ringmagnet 20.2 angeordnet. Der Absatz 22.4 nimmt den Ringmagneten 20.2 derart auf, dass dessen Außenfläche bündig mit der Mantelfläche des zylinderförmigen Abschnittes 22.2 abschließt. Dieser bildet in Verbindung mit dem im Lagerungsäbschnitt 19.9 des Kupplungsgehäuses 19 entsprechend ihn umgebenden äußeren Ringmagneten 20.1 ein Magnetringlager 20.3 aus.

Im Magnetringlager 20.3 sind die beiden Ringmagnete 20.1, 20.2 derart angeordnet, dass z.B. der Nordpol des äußeren Ringmagnet nach distal und der Südpol nach proximal ausgerichtet ist. Der Nord- und der Südpol des inneren Ringmagneten sind entsprechend entgegengesetzt ausgebildet. Entsprechend können die Nord- und Südpole der beiden Ringmagneten auch umgekehrt angeordnet sein. Das Magnetringlager 20.3 zentriert die Antriebswelle 4 in axialer und in radialer Richtung. Die radiale Zentrierung erfolgt durch die magnetischen Anziehungskräfte in radialer Richtung. Die axiale Zentrierung erfolgt dadurch, dass bei einem kleinen Versatz des inneren Ringmagneten 20.2 magnetische Rückstellkräfte erzeugt werden, die den inneren Ringmagneten 20.2 in eine in Axialrichtung mit der Position des äußeren Ringmagneten 20.1 übereinstimmende Stellung ziehen. Bei einem größeren Versatz treten hingegen Abstoßungskräfte zwischen den beiden Magnetringen 20.1 und 20.2 auf, wodurch sie auseinander gedrückt werden.

Im Magnetringlager 20.3 berühren sich die Ringmagneten 20.1, 20.2 nicht, d.h. es ist keine Schmierung erforderlich. Zudem wirkt das Magnetringlager schwingungsdämpfend.

Am proximalen Ende des Kupplungselements ist im scheibenförmigen Abschnitt 22.3 des Magnetkupplungselements 22 eine Magnetaufnahme 22.5 ausgebildet. Die Magnetaufnahme 22.5 ist eine zentrische zirkuläre Ausfräsung.

Die zentrische zirkuläre Ausfräsung 22.5 weist einen Durchmesser von in etwa 16,5 mm und eine Tiefe von in etwa 3 mm auf.

Die Magnetaufnahme 22.5 nimmt die aus vier Segmenten bestehende ringförmige distale Magneteinheit 23.1 auf. Die ringförmige distale Magneteinheit ist in die Magnetaufnahme 22.5 eingeklebt.

In der proximalen Stirnseite des Kupplungselements 22 ist mittig eine Kugelkopflageraufnahme 22.6 ausgebildet. Die Kugelkopflageraufnahme 22.6 ist eine etwa halbkugelförmige Ausnehmung 22.6.

Die halbkugelförmige Ausnehmung 22.6 weist einen Durchmesser von in etwa 0,5 bis 1,3 mm auf.

Die Vierkantstange 21 bzw. der zylinderförmige Abschnitt des Kupplungselements 22 wird vom vierten Bohrungsabschnitt 19.8 bzw. vom Lagerungsabschnitt 19.9 des Kupplungsgehäuses 19 aufgenommen. Der scheibenförmige Abschnitt 22.3 des Kupplungselements 22 wird vom distalen Kupplungsabschnitt 19.10 des Kupplungsgehäuses 19 aufgenommen.

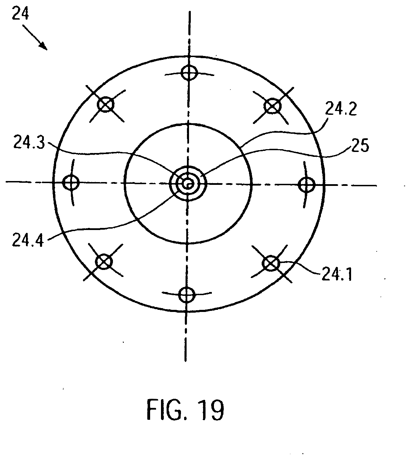

Das Kupplungsgehäuse 19 wird durch eine Abschlussscheibe 24 hermetisch von der Motoranordnung getrennt (Fig. 19). Das Kupplungsgehäuse 19 ist bis auf die Spülbohrung 19.15 im Kupplungsgehäuse 22 und die Freiräume zwischen dem Antriebswellendurchtrittsabschnitt 19.7 und der Antriebswelle 4 gas- und flüssigkeitsdicht.

Die Abschlussscheibe 24 ist auf dem Absatz 19.12 des Kupplungsgehäuses 19 angeordnet und ist mittels acht Schrauben fixiert, die entsprechend von radiär symmetrisch in der Abschlussscheibe 24 angeordneten Bohrungen 24.1 aufgenommen sind und in den Gewindebohrungen 19.13 des Kupplungsgehäuses 19 verschraubt sind. Diese Verbindung ist flüssigkeits- und gasdicht ausgebildet. Die Abschlussscheibe 24 ist beispielsweise aus Polymethylacrylat (PMMA) oder einem anderen nicht metallischen Material (wie z.B. Peek, PEBAX, Teflon, PP, PE, alle spritzgießbaren, extrudierbaren oder zu spanenden, nicht-magnetischen Materialien) ausgebildet.

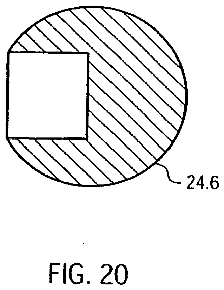

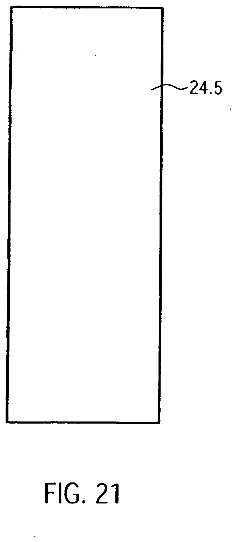

Distalseitig weist die Abschlussscheibe 24 eine zentrale Verdickung 24.2 auf. Im Zentrum der Abschlussscheibe 24 ist eine Durchgangsbohrung 24.3 und eine zentrische halbkugelige Ausfräsung 24.4 ausgebildet. In der Durchgangsbohrung 24.3 ist ein zylinderförmiger Zentrierstift 24.5 fixiert (Fig.21). Auf dem Zentrierstift 24.5 ist ein Kugelkopf 24.6 angeordnet der in der halbkugeligen Ausfräsung aufgenommen ist (Fig. 15, Fig. 20).

Die distale Magneteinheit 23.1 wird nach proximal mit einer Kraft beaufschlagt. Diese entgegengesetzten Kräfte bewirken eine resultierende Kraft, mit der das Kupplungselement 22 gegen den Kugelkopf 24.6 gedrückt wird. Diese resultierende Kraft wird so eingestellt, dass der Kugelkopf 24.6 sicher gelagert ist und dennoch der Verschleiß im Kugelkopflager gering gehalten wird.

Der Kugelkopf 24.6 bildet in Verbindung mit der distal angeordneten Kugelkopflageraufnahme 22.7 des Kupplungselements 22 ein Kugelkopflager 25 aus. Das Kugelkopflager 25 ist ein Gleitlager. Es sind jedoch auch andere Gleitlager, wie z.B. ein Kegelkopflager bzw. ein Zylinderkopflager möglich, bei welchen an Stelle der Kugel ein Kegel bzw. ein Zylinder als Lagerkörper vorgesehen ist. Die Aufnahme ist der Form des Lagerkörpers entsprechend angepasst.

Das Kugelkopflager 25 stellt in Verbindung mit dem Magnetringlager 20.3 eine axiale Laufzentrierung und -führung des Kupplungselements 22 und der darin angeordneten Antriebswelle 4 innerhalb des Kupplungsgehäuses 19 sicher.

Die axiale Zentrierung des Magnetringlagers 20.3 erfolgt dadurch, dass der innere Ringmagnet 20.2 in Axialrichtung nicht genau mittig im äußeren Ringmagneten 20.1 angeordnet ist, sondern leicht nach proximal vesetzt ist. Dadurch wird der innere Ringmagnet 20.2 nach distal mit einer Kraft beaufschlagt. Der Kugelkopf 24.6 kann aus Rubin, Aluminiumoxid oder einem harten Kunststoff ausgebildet sein.

Um zu verhindern, dass Blut und Serum durch die Freiräume zwischen der Antriebswelle 4 und dem proximalen Rotorlager 17.2, aufgrund der Rotationsbewegung der Antriebswelle 4, angesaugt werden und das Blut gerinnt und/oder an der Antriebswelle 4 anhaftet, wird über die Spülbohrung im Kupplungsgehäuse ein Spülmedium eingebracht um einen Gegendruck zum angesaugten bzw. hineingedrückten Blutstrom zu erzeugen. Dadurch wird das Kugelkopflager geschmiert. Geeignete Spülmedien sind z.B.:
- 3-20% Gkukoselösung;
- 5-40% Dextranlösung mit Molmasse 5.000 bis 65.000, insbesondere 10% Dextranlösung MM 40.000 in 0,9% NaCl;
- Ringerlösung: eine Elektrolytgemischlösung mit K, Na, Mg;
- andere physiologische Elektrolytlösungen.

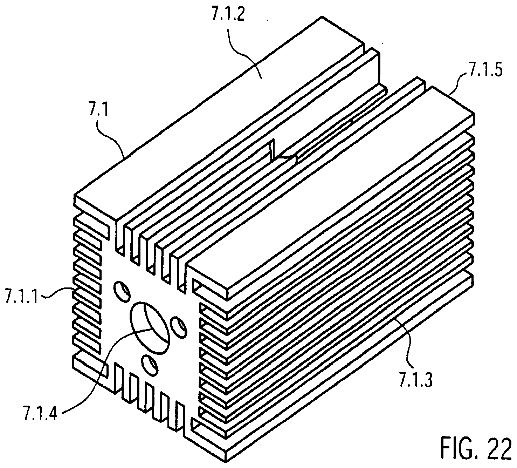

Die Motoranordnung umfasst die proximale Magneteinheit 23.2, eine proximale Magnetaufnahme 26, einen Kupplungsflansch 27, eine Motoraufnahme 7.1, mit einem darauf angeordneten Kühllüfter und den Motor 7 (Fig. 14, Fig. 22).

Auf der proximalen Seite der Abschlussscheibe 24 ist in einem Abstand von in etwa 0,5 bis 8 mm und vorzugsweise etwa 1 bis 2 mm eine proximale Magneteinheit 23.2 axial fluchtend zur distalen Magneteinheit 23.1 angeordnet. Die proximale ringförmige Magneteinheit 23.2 weist analog zur distalen Magneteinheit 23.1 vier Segmente auf.

Die Magnetaufnahme 26 ist scheibenförmig ausgebildet und weist auf ihrer distalen Seite eine zentrische zirkuläre Ausfräsung 26.1 auf. In die Ausfräsung 26.1 sind analog zur distalen Magneteinheit 23.1 vier Magnetsegmente mittels Zwei-Komponenten-Epoxydharzkleber oder Cyanacrylatkleber eingeklebt (s.o.).

Die vier Segmente der distalen und der proximalen Magneteinheit 23.1, 23.2 können als gebogene Stabmagneten ausgebildet sein die an ihren Endbereichen jeweils eine unterschiedliche Polung aufweisen. Die vier Segmente können auch als vier Viertel eines gebogenen Ringmagneten ausgebildet sein. Die Segmente können auch als kurze, axial ausgerichtete Stabmagnete ausgebildet sein, die ringförmig angeordnet sind. Es können auch mehr als vier Segmente vorgesehen sein. In der Ausgangstellung sind die beiden Magneten derart angeordnet, dass sich jeweils ein Nord- und ein Südpol der Stabmagneten der beiden Magneteinheiten 23.1, 23.2 überlagern und sich gegenseitig anziehen.

Die vier Segmente sind viermal mit ihren Nord- und Südpolen alternierend auf Stoß angeordnet, so dass sich die Segmente einer Magneteinheit anziehen. Die distale und der proximale Magneteinheit 23.1, 23.2 sind zueinander derart angeordnet, dass jeweils komplementäre Pole zueinander gegenüberliegend angeordnet sind. Hierdurch ziehen sich die beiden Magneteinheiten an und es lässt sich ein Drehmoment übertragen, da die Magnetkräfte diese komplementäre Polanordnung beibehalten möchte.

Die zentrische zirkuläre Ausfräsung 26.1 weist einen Durchmesser von in etwa 16,5 mm und eine Tiefe von in etwa 3 mm auf.

Die Magnetaufnahme 26 ist mit einer Motorwelle 7.2 des Motors 7 verbunden. Die Magnetaufnahme 26 ist drehbar innerhalb einer entsprechend ausgeformten Aussparung des Kupplungsflansches 27 der Motoraufnahme angeordnet. Entlang des äußeren Umfangs des ringförmigen Steges der Aussparung sind gleichmäßig beabstandet drei Passstifte 27.1 angeordnet.

Über die L-förmigen Ausfräsungen 19.14 des Kupplungsgehäuses 19 wird das Kupplungsgehäuse 19 mit den Passstiften 27.1 des Kupplungsflansches 27 der Motoranordnung verbunden.

Der Kupplungsflansch 27 ist unter Wahrung der Achssymmetrie auf einer distalen Stirnseite 7.1.1 der Motoraufnahme befestigt. Die Motoraufnahme 7.1 ist ein quaderförmiger Körper, an dessen Seitenflächen 7.1.2 Kühllamellen 7.1.3 angeordnet sind.

Die Motoraufnahme 7.1 weist in axialer Richtung eine mittig angeordnete Bohrung 7.1.4 auf. Durch diese Bohrung 7.1.4 ist die Motorwelle 7.2 geführt. Weiterhin ist eine axial fluchtende Ausnehmung 7.1.5 vorgesehen in der der Motor 7 angeordnet ist. Der Motor 7 ist beispielsweise ein Standard-Elektromotor der Firma Faulhaber mit einer Leistung von 38 W bei 30000 U/min oder ein anderer geeigneter Motor.

An einer Seitenfläche 7.1.2 der quaderförmigen Motoraufnahme 7.1 ist ein Kühlungslüfter angeordnet.

Über dem Pumpenkopf 3 und einem distalen Bereich des proximalen Katheterschaftstücks ist ein Deckschlauch 29 angeordnet. Der Deckschlauch 29 weist einen Innendurchmesser auf, der im Bereich des Pumpenkopfes 3 dem Außendurchmesser des nicht expandierten Pumpengehäuses entspricht. Der Außendurchmesser des Deckschlauches beträgt in etwa 3 mm.

Nachfolgend wird das Verfahren zum Kuppeln mit der Magnetkupplung 9 beschrieben.

Die beiden Magneteinheiten 23.1, 23.2 sind durch die Abschlussscheibe 24 im Kupplungsgehäuse 19 räumlich voneinander getrennt. Durch die magnetischen Anziehungskräfte zwischen den beiden Magneteinheiten 23.1, 23.2 erfolgt eine kraftschlüssige Verbindung. Hierbei stehen sich jeweils entgegengesetzte Pole der beiden Magneteinheiten 23.1, 23.2 gegenüber, wodurch sie sich anziehen und ein drehfester Kraftschluß gebildet wird.

Weiterhin wird hierdurch die Kugelkopflageraufnahme 22.7 des Kupplungselements 22 auf den Kugelkopf 24.6 der Abschlussscheibe 24 gepresst und bildet das Kugelkopflager 25 aus. Das Kugelkopflager zentriert den axialen Lauf der Antriebswelle 4.

Durch die Anordnung der beiden Ringmagnete 20.1, 20.2 des Magnetringlagers 20.3 wird der innere Ringmagnet 20.1 mit konstantem Abstand im äußeren Ringmagneten 20.2 radial geführt. Auf diese Weise zentriert und führt das Magnetringlager 20.3 in Verbindung mit dem Kugelkopflager 25 den rotationssymmetrischen Lauf des Kupplungselements 22 bzw. der Antriebswelle 4, um Schläge bzw. eine Unwucht zu verhindern.

Über die kraftschlüssige Verbindung zwischen den Magneteinheiten 23.1, 23.2 wird die vom Motor 7 über die Motorwelle 7.2 auf die proximale Magneteinheit 23.2 übertragene Drehbewegung auf die distale Magneteinheit 23.1 überführt.

Die Motorwelle 7.2 dreht sich mit einer Drehzahl von in etwa 20000 U/min bis 40000 U/min und vorzugsweise etwa 32000 U/min bis 35000 U/min, die auf die Antriebswelle 4 übertragen werden. Bei 32000 U/min ergibt sich eine Förderleistung des Rotors 3.2 von in etwa 2 l/min bis 2,5 l/min bei einem Differenzdruck von 60 mm Hg.

Im Falle einer Blockierung des Rotors 3.2 muss die kraftschlüssige Verbindung zwischen Motor 7 und Antriebswelle 4 getrennt werden, um ein "Aufwickeln" der Antriebswelle 4 bei feststehendem Rotor zu verhindern. Durch ein "Aufwickeln" der Antriebswelle 4 könnte der Pumpenkopf 3 seine Position verändern und könnte dadurch das Herz und/oder die Aorta bzw. die Vene beschädigen.

Sobald der Rotor 3.2 blockiert, verdreht bzw. verkürzt sich die Antriebswelle 4, der Widerstand an der distalen Magneteinheit 23.1 steigt. Die Magnetfelder zwischen der proximalen und der distalen Magneteinheit 23.2, 23.1 überlagern sich im Betrieb nicht vollständig, da die distale Magneteinheit 23.1 der proximalen Magneteinheit 23.2 immer ein wenig nachläuft. Erhöht sich nun das benötigte Drehmoment an der distalen Magneteinheit 23.1 überlagern sich die Nord- und Südpole der Magneteinheiten 23.1, 23.2 nicht mehr sondern stoßen einander ab. Hierdurch wird die distale Magneteinheit 23.1 von der proximalen Magneteinheit 23.2 in Richtung distal gedrückt. Die magnetische Verbindung zwischen den beiden Magneteinheiten 23.1, 23.2 ist getrennt. Die Antriebswelle 4 steht sofort still.

Durch die Verschiebung des Kupplungselements 22 in Richtung distal wird der innere Ringmagnet 20.2 des Kupplungselements 22 ebenfalls in Richtung distal verschoben und die Nord- und Südpole der beiden Ringmagneten 20.1, 20.2 des Magnetringlagers 20.3 überlagern sich nicht mehr, sondern stoßen sich ab. Hierdurch wird die Kupplung 9 im entkoppelten Zustand gehalten und es kommt zu einer dauerhaften Entkopplung von Motor 7 und Antriebswelle 4.

Durch das Magnetringlager 20.3 bzw. die magnetische Verbindung der beiden Magneteinheiten 23.1, 23.2 wird der Betrag des übertragbaren Drehmoments begrenzt. Sobald das eingestellte Drehmoment überschritten wird, trennen sich die beiden Magneteinheiten 23.1, 23.2. Die distale Magneteinheit 23.1 kann der proximalen Magneteinheit 23.2 aufgrund der schnellen Drehbewegung nicht mehr nachlaufen, da die magnetischen Bindungskräfte nicht mehr ausreichen. Hierdurch überlagern sich die Nord- und Südpole nicht mehr und die Magneteinheiten 23.1, 23.2 stoßen sich ab. Die Verbindung der Magneteinheiten 23.1, 23.2 wird getrennt und das maximal übertragbare Drehmoment begrenzt. Die Magneteinheiten 23.1, 23.2 werden vom Magnetringlager 20.3 durch die gegenseitige Abstoßung der Ringmagnete 20.1, 20.2 im entkoppelten Zustand gehalten.

Dieser Zustand lässt sich durch das Ansetzen eines äußeren Magnetfelds wieder ändern. Durch einen von distal nach proximal am Kupplungsgehäuse 19 vorbeigeführten Magneten können die beiden Magneteinheiten 23.1, 23.2 wieder in ihre gekoppelte Ausgangsstellung gebracht werden.

Erfindungsgemäß sind das Kupplungsgehäuse 19 und die Motoranordnung 30 räumlich voneinander getrennt. Hierdurch ist es möglich, die Antriebswelle 4 über die an der Spülbohrung 19.15 angeordnete Pumpe trotz der hohen Drehzahl mit in etwa 5-10 ml/h zu schmieren um so die Reibung zu minimieren. Es kann auch vorgesehen sein eine Infusion über die Spülbohrung 19.15 einzubringen, die die Antriebswelle 4 ebenfalls schmiert.

Der kleine Durchmesser der Antriebswelle ist bei hohen Drehzahlen von in etwa 32000 U/min von Vorteil. Bei größeren Durchmessern würde die Umfangsgeschwindigkeit zu hoch werden und es könnte aufgrund der Reibung zu einer Beschädigung der Antriebswelle 4 bzw. der daran angrenzenden Bauteile kommen.

Aufgrund der räumlichen Trennung durch die Abschlussscheibe 24 ist es möglich, die Antriebswelle 4 zu schmieren bzw. abzudichten. Kein bekanntes Lager, durch das eine Welle hindurchgeführt ist, würde bei dieser Größe und derartigen Drehzahlen dicht bleiben und einen einwandfreien Lauf ermöglichen.

Aus der Anordnung des Kugelkopflagers 25 (Gleitlager), des Magnetringlagers 20.3 (berührungsfrei, dämpfend und zentrierend) und des axialen Gleitlagers zwischen Antriebswelle 4 und Kupplungsgehäuse 19 ergeben sich drei Stabilisationspunkte. Dadurch kann die Antriebswelle 4 ein Drehmoment auch bei einer axialen Längenveränderung übertragen (Verlängerung und Verkürzung). Zu einer Längenänderung kommt es beispielsweise, wenn der Pumpenkopf 3 komprimiert wird. Dabei wird der Rotor 3.2 zusammengedrückt, um die Antriebswelle gefaltet und im Gehäuse festgeklemmt. Das Pumpengehäuse 3.1 verlängert sich proximal. Die Antriebswelle 4 kann sich soweit bewegen, daß sie nicht vom Rotor 3.2 abgerissen wird. Durch die Verschiebbarkeit der Antriebswelle 4 kann die Längenänderung des PU-Katheterschafts aufgrund von Flüssigkeitsaufnahme, Temperaturunterschieden, und durch Biegung des Katheterschaftes 8.2, die die Längenverhältnisse zwischen Antriebswelle 4 und Katheterschaft 8.2 beeinflussen, ausgeglichen werden.

Dieser Mechanismus ist durch die Verschiebbarkeit der Vierkantstange 21 innerhalb der axialen Ausnehmung 22.1 möglich.

Der Pumpenkopf 3 wird derart in der linken Herzkammer angeordnet, dass der Abströmschlauch 18 in etwa mittig im Übergang der Aorta zum Herzen, also im Bereich der Herzklappe angeordnet ist. Die Katheter-Vorrichtung 1 ist vorzugsweise derart ausgebildet, dass mit ihr ein bestimmter Pumpendruck im Bereich von etwa 100 mm Hg bis 150 mm Hg erzeigt werden kann. Befindet sich das Herz in der Systole, dann fördert die Katheter-Vorrichtung Blut, wenn der vom Herz aufgebaute Druck kleiner als der Pumpendruck ist. Ein krankes Herz wird somit entlastet. Während der Diastole liegt eine entgegengesetzte Druckdifferenz vor. Ist die Druckdifferenz größer als der Pumpendruck, dann kann die Katheter-Vorichtung kein Blut fördern. Hierbei wird der Abströmschlauch von der Herzklappe zusammengedrückt, so dass er abgedichtet ist. Ist die Druckdifferenz jedoch kleiner als der Pumpendruck, dann wird etwas Blut entgegen der Druckdifferenz gefördert.

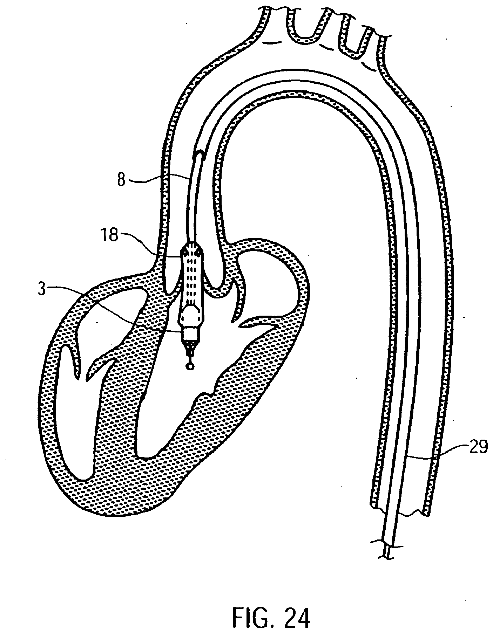

Fig. 24 zeigt die positionierte Katheter-Vorrichtung 1 zur Linksherz-Unterstützung. Der Pumpenkopf 3 befindet sich vollständig in der linken Herzkammer. Der Abströmschlauch erstreckt sich durch die Herzklappe.

Zum Einbringen der Katheter-Vorrichtung wird zunächst ein Deckschlauch 29 mittels eines Führungsdrahtes bis in die linke Herzkammer geführt (Seldinger-Technik). Der Führungsdraht wird dann aus dem Deckschlauch entfernt. Die Katheter-Vorrichtung 1 wird mit komprimierten und gekühlten Pumpengehäuse 3.1 und Rotor 3.2 durch den Deckschlauch eingeführt, bis die Katheter-Vorrichtung 1 mit dem Pumpenkopf 3 die linke Herzkammer erreicht hat. Die Entfaltung erfolgt, indem der Deckschlauch 29 auf dem fixierten Katheterschaft 8 zurückgezogen wird, bis die Spitze des Deckschlauchs 29 den Pumpenkopf freigegeben 3 hat.

Zur Entfernung des Systems wird der Deckschlauch 29 bis zur Schaftkappe 10 vorgeschoben, wodurch Rotor 3.2 und Pumpengehäuse 3.1 im komprimierten Zustand in den Deckschlauch 29 eingezogen werden, wonach dieser durch die Punktionsstelle hindurch extrahiert wird.

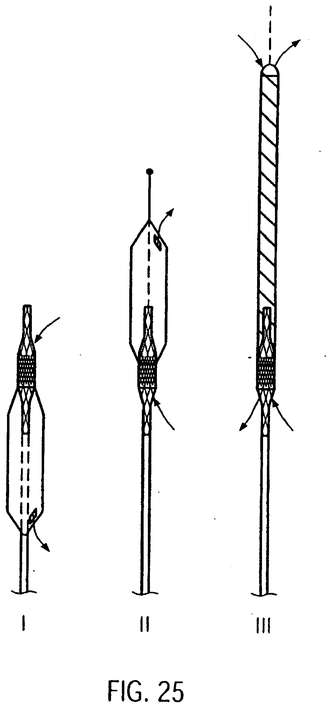

In einem weiteren Ausführungsbeispiel der vorliegenden Erfindung ist vorgesehen ein Pumpmedium von proximal nach distal, das heißt entgegen der ursprünglichen Förderrichtung 5 zu pumpen (Fig. 25 II). Um den Rotor 3.2 in axialer Richtung zu lagern und die Lagerkräfte aufzunehmen ist die Lagerscheibe 15 auf der proximalen Seite des Rotors 3.2 angeordnet. Die Förderrichtung nach distal kann entweder durch Umkehren der Drehrichtung gegenüber obigem Ausführungsbeispiel oder durch Umkehren der Steigung des Rotors 3.2 realisiert werden. Der Abströmschlauch 18 ist am distalen Ende des Pumpenabschnitts des Pumpengehäuses 19 angeordnet und erstreckt sich in distale Richtung über den Pumpenkopf hinaus. Zur Aussteifung des Abströmschlauchs 18 kann der Abströmschlauch eine Gitterstruktur aus einem Formgedächtnismaterial, z.B. ähnlich zu der des Pumpengehäuses, aufweisen.Die Schaftkappe 10 erstreckt sich bis über das distale Ende des Abströmschlauches.

Beim Betrieb strömt das Pumpmedium durch die nun als Einlass dienende Auslassöffnungen des Pumpengehäuses ins Pumpengehäuse und gelangt über die nun als Auslass dienende Einlassöffnung des Pumpengehäuses in den Abströmschlauch 18. Über das distale Ende des Abströmschlauchs tritt das Pumpmedium aus der Katheter-Vorrichtung 1 aus.

Das eben beschriebene Ausführungsbeispiel kann zum Beispiel für den Einsatz im rechten Ventrikel vorgesehen sein.

In einem weiteren Ausführungsbeispiel kann die erfindungsgemäße Katheter-Vorrichtung auch derart ausgebildet sein, dass ein Pumpen von distal nach proximal und von proximal nach distal möglich ist (Fig. 25 III).

Bei diesem Ausführungsbeispiel sind Lagerscheiben 15 am distalen und am proximalen Ende des Rotors 3.2 vorgesehen. Der Abströmschlauch 18 ist am distalen Ende des Pumpenabschnittes 3.1.3 des Pumpengehäuses 3.1 angeordnet und erstreckt sich in distaler Richtung. Der Abströmschlauch 18 weist zur Aussteifung eine Gitterstruktur, z.B. ähnlich dem Pumpengehäuse auf. Die Gitterstruktur ist mit einer PU-Haut bespannt. Der Durchmesser des Abströmschlauchs entspricht in etwa dem des expandierten Pumpengehäuses.

Beim Betrieb kann ein Pumpmedium durch die Auslassöffnungen des Pumpengehäuses ein- bzw. austreten. Das Pumpmedium tritt dann beispielsweise über die Auslassöffnungen des Pumpengehäuses und die Einlassöffnungen des Pumpengehäuses in den Abströmschlauch und tritt am distalen Ende des Abströmschlauchs aus. Bei umgekehrter Pumprichtung erfolgt die Durchströmung der Katheter-Vorrichtung entsprechend umgekehrt. Das heißt, dass das Pumpmedium am distalen Ende des Abströmschlauchs in den Abströmschlauch eintritt und über die Einlassöffnung des Pumpengehäuses zu den Auslassöffnungen des Pumpengehäuses gelangt. Somit ist durch den druck- und sogstabilisierten Abströmschlauch 18 ein Abströmen nach distal oder proximal möglich.

Das eben beschriebene Ausführungsbeispiel kann beispielsweise für Drainagen oder zum Auffüllen von Hohlorganen bzw. -räumen verwendet werden.

Die Umkehr der Förderrichtung kann einerseits durch Umkehren der Drehrichtung des Rotors und andererseits durch Umkehrung der Steigung des Rotors erzielt werden.

Die Erfindung ist oben anhand eines Ausführungsbeispiels beschrieben, bei dem die Magneteinheiten jeweils vier gebogene Stabmagnete aufweisen, die jeweils mit entgegengesetzten Polen aneinander gesetzt sind. Im Rahmen der Erfindung können die Magneteinheiten auch derart ausgebildet sein, dass die Nord- und Südpole der Magneteinheiten in axialer Richtung ausgerichtet sind, wobei die Pole an den axial nach distal bzw. proximal weisenden Flächen angeordnet sind. Die Magnete sind entsprechend den vorherigen Ausführungsbeispielen ringförmig angeordnet.

Durch eine derartige Ausrichtung der Nord- und Südpole der Magnete ziehen sich die beiden Magneteinheiten mit höheren Magnetkräften an. Dadurch ist möglich, ein höheres Drehmoment über die Kupplung zu übertragen.

Eine derartige Kupplung kann beispielsweise zum Antreiben eines Fräskopfs anstelle eines Rotors verwendet werden. Mit einer solchen Mikrofräse können z.B. Nierensteine oder Knochen minimal-invasiv gefräst werden.

Die Anzahl der Magnete kann grundsätzlich beliebig variiert werden.

Die radiale Komprimierbarkeit der Komponenten erlaubt die Realisierung eines für eine perkutane Implantation in Seldinger-Technik vertretbar kleinen Punktionsdurchmessers, aufgrund des sehr geringen Durchmessers der Katheter-Vorrichtung von etwa 3 mm. Durch die Expansion des Rotors bis zu einem Durchmesser von in etwa 15 mm ist es dennoch möglich sehr hohe Förderleistungen zu realisieren.

Aus dem Stand der Technik sind aufweitbare Katheterpumpen (z.B. US 4 753 221) bekannt die einen Propeller mit mehreren steifen Pumpenflügeln aufweisen. Diese sind schwenkbar angeordnet. Da die Flügel steif sind können sie nicht beliebig breit ausgebildet werden, da sie hierdurch im zusammengeklappten Zustand dem Katheter eine zu große Dicke verleihen würden. Daher ist die Förderleistung begrenzt.

Der Rotor nach der WO 99/44651 weist ein elastisches Band auf, um die Enden einer Nitinolwendel mit einer Rotationsachse zu verbinden. Durch diese elastische Verbindung ist die Wendel nicht perfekt zentriert. Dadurch kommt es beim Pumpen zu Schwingungen, die höhere Drehzahlen bzw. Förderleistungen unmöglich machen.

Durch die Rahmenstruktur des Rotors mit Begrenzungsrahmen und Rotorstreben gemäß der Katheter-Vorrichtung 1 ist der Rotor stabiler, faltbar und lässt sich auf nahezu beliebig große Durchmesser expandieren. Dadurch dass eine nahezu beliebig lange Ausbildung des Rotors in Längsrichtung möglich ist, ist die radiale Erstreckung des Rotors frei wählbar. Somit können beliebige insbesondere sehr hohe Förderleistungen erzielt werden und es ist möglich, die Förderleistung für jeden Anwendungsfall individuell anzupassen.

Die Steigung des Rotors lässt sich ebenfalls beliebig variieren. Der Rotor kann mit einem oder mehreren Rotorblättern ausgebildet sein, wobei die Rotorblätter entsprechend eine viertel, eine halbe, eine ganze oder beliebig viele Umschlingungen um die Antriebswelle aufweisen. Das heißt, dass der erfindungsgemäße Rotor ist in seiner Größe, seiner Form, seiner Steigung beliebig variierbar ist und daher für die verschiedensten Anwendungen genutzt werden kann.

**Bezugszeichenliste**

| | | | |
|---|---|---|---|
| 1 | Katheter-Vorrichtung | 7.1.2 | Seitenfläche |
| 2 | distales Ende | 7.1.3 | Kühllamellen |
| 3 | Pumpenkopf | 7.1.4 | Bohrung |
| 3.1 | Pumpengehäuse | 7.1.5 | Ausnehmung |
| 3.1.1 | distaler Verbindungsabschnitt | 7.2 | Motorwelle |
| 3.1.2 | Ansaugabschnitt | 8. | Katheterschaft |
| 3.1.3 | Pumpenabschnitt | 8.1 | distales Katheterschaftstück |
| 3.1.4 | Auslassabschnitt | 8.2 | proximales Katherschaftstück |
| 3.1.5 | proximaler Verbindungsabschnitt | | |
| | | 9. | Kupplung |
| 3.1.6 | Gitterstruktur | 10. | Schaftkappe |
| 3.1.7 | Öffnungen | 10.1 | Kugel |
| 3.1.7.1 | kleine Raute | 10.2 | zylinderförmiger Abschnitt |
| 3.1.7.2 | große Raute | 10.3 | Durchgangsbohrung |
| 3.1.7.3 | mittelgroße Raute | 10.4 | axiale Bohrung |
| 3.1.8 | PU-Bespannung vom Pumpengehäuse | 10.5 | Stufe |
| | | 12.1 | distale Verbindungsbuchse |
| 3.2 | Rotor | 12.2 | proximale Verbindungsbuchse |
| 3.2.1 | Rahmenstruktur | 13.1 | distaler Wellenschutz |
| 3.2.2 | Begrenzungrahmen | 13.2 | proximaler Wellenschutz |
| 3.2.3 | Rotorstreben | 14 | Führungsspirale |
| 3.2.4 | Ringe | 15 | Lagerscheibe |
| 4. | Antriebswelle | 15.1 | Durchgangsbohrung |
| 4.1 | distaler Abschnitt der Antriebswelle | 16 | Abstandshülsen |
| | | 17 | distales Rotorlager |
| 4.2 | Pumpenabschnitt der Antriebswelle | 18 | Abströmschlauch |
| | | 18.1 | Auslassöffnung |
| 4.3 | proximaler Abschnitt der Antriebswelle | 19 | Kupplungsgehäuse |
| 5. | Förderrichtung | 19.1 | distaler zylinderförmiger Abschnitt |
| 6. | proximales Ende | 19.2 | konisch aufweitender Abschnitt |
| 7. | Motor | 19.3 | zweiter zylinderförmiger Abschnitt |
| 7.1 | Motoraufnahme | 19.4 | proximaler zylinderförmiger Abschnitt |
| 7.1.1 | Stirnseite | | |
| 19.5 | Katheterschaftaufnahmeabschnitt | 22.3 | scheibenförmiger Abschnitt |
| | | 22.4 | Absatz |
| 19.6 | Führungsspiraleaufnahmeabschnitt | 22.5 | Magnetaufnahme |
| | | 22.6 | Kugelkopflageraufnahme |
| 19.7 | Antriebswellendurchtrittsabschnitt | 23.1 | distale Magneteinheit |
| | | 23.2 | proximale Magneteinheit |
| 19.8 | vierter Bohrungsabschnitt | 24 | Abschlussscheibe |
| 19.9 | Lagerungsabschnitt | 24.1 | Bohrungen |
| 19.10 | distaler Kupplungsabschnitt | 24.2 | Verdickungen |
| 19.11 | proximaler Kupplungsabschnitt | 24.3 | Durchgangsbohrung |
| 19.12 | Absatz | 24.4 | halbkugelige Ausfräsung |
| 19.13 | Gewindebohrung | 24.5 | Zentrierstift |
| 19.14 | L-förmige Ausfräsung | 24.6 | Kugelkopf |
| 19.15 | Spülbohrung | 25 | Kugelkopflager |
| 20.1 | äußerer Ringmagnet | 26 | Magnetaufnahme |
| 20.2 | innerer Ringmagnet | 26.1 | Ausfräsung |
| 20.3 | Magnetringlager | 27 | Kupplungsflansch |
| 21 | Vierkantstange | 27.1 | Passstifte |
| 21.1 | Ausnehmung | 28 | |
| 22 | Kupplungselement | 29 | Deckschlauch |
| 22.1 | Ausnehmung | 30 | Motoranordnung |
| 22.2 | zylinderförmiger Abschnitt | | |

## Patentansprüche

1. Katheter-Vorrichtung, umfassend:
- eine Antriebswelle (4),
- einen Rotor (3.2), der mit der Antriebswelle (4) verbunden ist, indem er auf der Antriebswelle (4) befestigt ist, wobei der Rotor (3.2) derart ausgebildet ist, dass sich der Rotor (3.2) nach einer aufgezwungenen Kompression radial entfaltet,
- einen an einem distalen Ende (2) der Katheter-Vorrichtung angeordneten Pumpenkopf (3),
- wobei der Pumpenkopf (3) umfasst:
- ein expandierbares und komprimierbares Gehäuse,
- mit einer rohrförmigen Gitterstruktur (3.1.6), mit Öffnungen (3.1.7), und
- Abschnitten, die zwischen einem distalen Ende (2) des Gehäuses (3.1) und einem proximalen Ende (6) des Gehäuses (3.1) angeordnet sind,
- wobei die Abschnitte in Förderrichtung (5) einen sich aufweitenden Ansaugabschnitt (3.1.2), einen Pumpenabschnitt (3.1.3) und einen sich verengenden Auslassabschnitt (3.1.4) umfassen, wobei der Pumpenabschnitt (3.1.3) den Rotor (3.2) aufnimmt,
- wobei die Gitterstruktur (3.1.6) des Gehäuses (3.1) im Pumpenabschnitt (3.1.3) mit einer elastischen Bespannung (3.1.8) derart bespannt ist, dass die Öffnungen (3.1.7) im Pumpenabschnitt (3.1.3) verschlossen sind,
**dadurch gekennzeichnet, dass**
- in der Gitterstruktur (3.1.6) zwischen dem Pumpenabschnitt (3.1.3) und dem Ansaugabschnitt (3.1.2) ein erster Übergangsbereich ausgebildet ist,
- wobei der erste Übergangsbereich Öffnungen umfasst, die größer sind als die Öffnungen im Pumpenabschnitt (3.1.3), und Öffnungen des Ansaugabschnitts (3.1.2) größer sind als die Öffnungen in dem ersten Übergangsbereich, und dass
- in der Gitterstruktur (3.1.6) zwischen dem Pumpenabschnitt (3.1.3) und dem Auslassabschnitt (3.1.4) ein zweiter Übergangsbereich ausgebildet ist,
- wobei der zweite Übergangsbereich Öffnungen umfasst, die größer sind als die Öffnungen im Pumpenabschnitt (3.1.3), und Öffnungen des Auslassabschnitts (3.1.4) größer sind als die Öffnungen in dem zweiten Übergangsbereich.

2. Katheter-Vorrichtung gemäß Anspruch 1, wobei die Öffnungen, die im Übergangsbereich vom Ansaugabschnitt zum Pumpenabschnitt angeordnet sind, zum Ansaugabschnitt (3.1.2) hin schrittweise zu größeren Öffnungen zusammengefasst sind.

3. Katheter-Vorrichtung gemäß einem der Ansprüche 1 bis 2, wobei das Gehäuse (3.1) von distal nach proximal folgende Abschnitte umfasst:
einen distalen Verbindungsabschnitt (3.1.1),
den Ansaugabschnitt (3.1.2),
den Pumpenabschnitt (3.1.3),
den Auslassabschnitt (3.1.4), und
einen proximalen Verbindungsabschnitt (3.1.5).

4. Katheter-Vorrichtung (1) gemäß Anspruch 3,
wobei die Öffnungen (3.1.7.1) im Pumpenabschnitt (3.1.3) kleiner sind als die Öffnungen (3.1.7.2) in den Verbindungsabschnitte (3.1.5, 3.1.1) und die Öffnungen (3.1.7.2) im dem distalen und dem proximalen Verbindungsabschnitt (3.1.1, 3.1.5) kleiner sind als die Öffnungen (3.1.7.3) im Einlassabschnitt (3.1.2) und im Auslassabschnitt (3.1.4).

5. Katheter-Vorrichtung gemäß einem der Ansprüche 1 bis 4 wobei die Öffnungen (3.1.7) als Polygone ausgebildet sind.

6. Katheter-Vorrichtung gemäß Anspruch 4, wobei die Öffnungen (3.1.7) als Rauten ausgebildet sind.

7. Katheter-Vorrichtung gemäß einem der Ansprüche 1 bis 6, wobei Gitterstreben der Gitterstruktur im Bereich der größeren Öffnungen in den Übergangsbereichen breiter als Gitterstreben der Öffnungen im Pumpenabschnitt (3.1.3) sind.

8. Katheter-Vorrichtung gemäß Anspruch 7, wobei die elastischen Bespannung Polyurethan, Polyethylen, Polypropylen, Silikon oder Parylene ist.

9. Katheter-Vorrichtung gemäß einem der vorhergehenden Ansprüche, wobei:
der Ansaugabschnitt (3.1.2) sich zum proximalen Ende des Gehäuses (3.1) hin konusförmig aufweitet, und
der Auslassabschnitt (3.1.4) sich zum proximalen Ende des Gehäuses (3.1) hin konusförmig verengt.

10. Katheter-Vorrichtung gemäß einem der Ansprüche 1 oder 2, umfassend
einen distalen Verbindungsabschnitt (3.1.1) zwischen dem distalen Ende (2) des Gehäuses und dem Ansaugabschnitt (3.1.2);
einen proximalen Verbindungsabschnitt (3.1.5) zwischen dem Auslassabschnitt (3.1.4) und dem proximalen Ende des Gehäuses;
wobei der distale und der proximale Verbindungsabschnitt (3.1.1, 3.1.5) Öffnungen (3.1.7.2) aufweisen, die größer als die Öffnungen (3.1.7.1) im Pumpenabschnitt (3.1.3) sind.

11. Katheter-Vorrichtung gemäß einem der vorhergehenden Ansprüche, wobei der Rotor (3.2) derart expandierbar und komprimierbar ist, dass der Rotor (3.2) im komprimierten Zustand einen Durchmesser aufweist, der etwa dem inneren Durchmesser des Pumpengehäuses (3.1) entspricht und der Rotor im expandierten Zustand etwa mit einem Abstand von zwischen 0,01 mm und 0,5 mm von dem inneren Durchmesser des Pumpengehäuses beabstandet ist.

12. Katheter-Vorrichtung nach einem der vorhergehenden Ansprüche, umfassend einen röhrenförmigen Deckschlauch (29), der den Pumpenkopf (3) in komprimiertem Zustand umschließt.

13. Katheter-Vorrichtung gemäß Anspruch 12, wobei das Pumpengehäuse (3.1) und der Rotor (3.2) derart ausgebildet sind, dass sie sich radial nach außen entfalten, nachdem der Deckschlauch (29) zurückgezogen wird.

14. Katheter-Vorrichtung gemäß Anspruch 12 oder 13, wobei der Rotor (3.2) und das Gehäuse (3.1) im komprimierten Zustand in den Deckschlauch (29) einfahrbar sind.

## Claims

1. A catheter device, comprising
- a drive shaft (4),
- a rotor (3.2), which is connected to the drive shaft (4), in that it is fixed on the drive shaft (4), wherein the rotor (3.2) is formed in such a way that the rotor (3.2) unfolds radially after an imposed compression,
- a pump head (3) arranged on a distal end (2) of the catheter device,
- the pump head (3) comprising:
- an expandable and compressible housing,
- comprising a tubular grid structure (3.1.6), comprising openings (3.1.7), and
- portions which are arranged between a distal end (2) of the housing (3.1) and a proximal end (6) of the housing (3.1),
- the portions comprising, as seen in the conveying direction (5), an expanding suction portion (3.1.2), a pump portion (3.1.3) and a narrowing outlet portion (3.1.4), the rotor (3.2) being housed in the pump portion (3.1.3),
- wherein the grid structure (3.1.6) of the housing (3.1), in the pump portion (3.1.3), is covered with an elastic covering (3.1.8) in such a way that the openings (3.1.7), in the pump portion (3.1.3), are closed,
**characterized in that**
- a first transition zone is formed in the grid structure (3.1.6) between the pump portion (3.1.3) and the suction portion (3.1.2),
- said first transition zone comprising openings which are larger than the openings in the pump portion (3.1.3), and openings of the suction portion (3.1.2) being larger than the openings in the first transition zone, and that
- a second transition zone is formed in the grid structure (3.1.6) between the pump portion (3.1.3) and the outlet portion (3.1.4),
- the second transition zone comprising openings which are larger than the openings in the pump portion (3.1.3), and openings of the outlet portion (3.1.4) being larger than the openings in the second transition zone.

2. The catheter device according to claim 1, wherein the openings which are arranged in the transition zone from the suction portion to the pump portion are combined in a stepwise manner to form larger openings towards the suction section (3.1.2).

3. The catheter device according to any of the claims 1 to 2, wherein the housing (3.1) comprises the following portions from distal to proximal:
a distal connecting portion (3.1.1),
the suction portion (3.1.2),
the pump portion (3.1.3),
the outlet portion (3.1.4), and
a proximal connecting portion (3.1.5).

4. The catheter device (1) according to claim 3,
wherein
the openings (3.1.7.1) in the pump portion (3.1.3) are smaller than the openings (3.1.7.2) in the connecting portions (3.1.5, 3.1.1,), and the openings (3.1.7.2) in the distal and the proximal connecting portion (3.1.1, 3.1.5) are smaller than the openings (3.1.7.3) in the inlet portion (3.1.2) and in the outlet portion (3.1.4).

5. The catheter device according to any of the claims 1 to 4, wherein the openings (3.1.7) are implemented as polygons.

6. The catheter device according to claim 4, wherein the openings (3.1.7) are implemented as rhombs.

7. The catheter device according to any of the claims 1 to 6, wherein grid struts of the grid structure in the area of the larger openings in the transition zones are broader than grid struts of the openings in the pump portion (3.1.3).

8. The catheter device according to claim 7, wherein the elastic covering is polyurethane, polyethylene, polypropylene, silicone or parylene.

9. The catheter device according to any of the preceding claims, wherein:
the suction portion (3.1.2) expands towards the proximal end of the housing (3.1) in a cone-shaped manner, and
the outlet portion (3.1.4) narrows towards the proximal end of the housing (3.1) in a cone-shaped manner.

10. The catheter device according to any of the claims 1 or 2, comprising
a distal connecting portion (3.1.1) between the distal end (2) of the housing and the suction portion (3.1.2);
a proximal connecting portion (3.1.5) between the outlet portion (3.1.4) and the proximal end of the housing;
the distal and the proximal connecting portion (3.1.1, 3.1.5) comprising openings (3.1.7.2) which are larger than the openings (3.1.7.1) in the pump portion (3.1.3).

11. The catheter device according to any of the preceding claims, wherein the rotor (3.2) is expandable and compressible in such a way, that the rotor (3.2) in the compressed state has a diameter which is approximately equal to the inner diameter of the pump housing (3.1) and in the expanded state is spaced from the inner diameter of the pump housing approximately by a distance of between 0.01 mm and 0.5 mm.

12. The catheter device according to any of the preceding claims, comprising a tubular cover hose (29) which encloses the pump head (3) in the compressed state.

13. The catheter device according to claim 12, wherein the pump housing (3.1) and the rotor (3.2) are designed such that they deploy radially outwards after the cover hose (29) has been pulled back.

14. The catheter device according to claim 12 or 13, wherein the rotor (3.2) and the housing (3.1) can be retracted into the cover hose (29) in the compressed state.

## Revendications

1. Dispositif de cathéter, comportant
- un arbre d'entraînement (4),
- un rotor (3.2), qui est connecté à l'arbre d'entraînement (4) en étant fixé sur l'arbre d'entraînement (4), le rotor (3.2) étant configuré de sorte que le rotor (3.2) s'étend radialement après une compression forcée,
- une tête de pompe (3) disposée à une extrémité distale (2) du dispositif de cathéter,
- la tête de pompe (3) comportant
- un boîtier extensible et compressible,
- avec une structure de grille tubulaire (3.1.6), avec des ouvertures (3.1.7),
- et des segments qui sont disposés entre une extrémité distale (2) du boîtier (3.1) et une extrémité proximale (6) du boîtier (3.1),
- les segments comportant, dans la direction de refoulement (5), un segment de succion (3.1.2) s'élargissant, un segment de pompe (3.1.3) et un segment de sortie (3.1.4) se rétrécissant, le segment de pompe (3.1.3) recevant le rotor (3.2),
- la structure de grille (3.1.6) du boîtier de pompe (3.1), dans le segment de pompe (3.1.3), étant recouverte d'un entoilage élastique (3.1.8) de sorte que les ouvertures (3.1.7), dans le segment de pompe (3.1.3), soient obturées.
**caractérisé par le fait que**
- dans la structure de grille (3.1.6) est réalisée, entre le segment de pompe (3.1.3) et le segment de succion (3.1.2), une zone de transition,
- la première zone de transition comportant des ouvertures qui sont plus grandes que les ouvertures dans le segment de pompe (3.1.3), et des ouvertures du segment de succion (3.1.2) étant plus grandes que les ouvertures dans la première zone de transition, et que
- dans la structure de grille (3.1.6) est réalisée, entre le segment de pompe (3.1.3) et le segment de sortie (3.1.4), une seconde zone de transition,
- la seconde zone de transition comportant des ouvertures qui sont plus grandes que les ouvertures dans le segment de pompe (3.1.3), et des ouvertures du segment de sortie (3.1.4) étant plus grandes que les ouvertures dans la seconde zone de transition,.

2. Dispositif de cathéter selon la revendication 1, dans lequel les ouvertures disposées dans la zone de transition du segment de succion au segment de pompe sont regroupées pour former, en étapes, des ouvertures plus grandes vers le segment de succion (3.1.2).

3. Dispositif de cathéter selon l'une des revendications 1 à 2, dans lequel le boîtier (3.1) comporte, du côté distal au côté proximal, les segments suivants :
un segment de connexion distal (3.1.1),
le segment de succion (3.1.2),
le segment de pompe (3.1.3),
le segment de sortie (3.1.4), et
un segment de connexion proximal (3.1.5).

4. Dispositif de cathéter selon la revendication 3, dans lequel
les ouvertures (3.1.7.1) dans le segment de pompe (3.1.3) et les ouvertures (3.1.7.2) dans les segments de connexion (3.1.5, 3.1.1) et les ouvertures (3.1.7.2) dans le segment de connexion distal et le segment de connexion proximal (3.1.1, 3.1.5) sont plus petites que les ouvertures (3.1.7.3) dans le segment d'entrée (3.1.2) et le segment de sortie (3.1.4).

5. Dispositif de cathéter selon l'une des revendications 1 à 4, dans lequel les ouvertures (3.1.7) sont réalisées en forme de polygones.

6. Dispositif de cathéter selon la revendication 4, dans lequel les ouvertures (3.1.7) sont réalisées en forme de losanges.

7. Dispositif de cathéter selon l'une des revendications 1 à 8, dans lequel les entretoises de grille de la structure de grille sont, à l'endroit des ouvertures plus grandes dans les zones de transition, plus larges que les entretoises de grille des ouvertures dans le segment de pompe (3.1.3).

8. Dispositif de cathéter selon la revendication 7, dans lequel l'entoilage élastique est en polyuréthane, en polyéthylène, en polypropylène, en silicone ou en parylène.

9. Dispositif de cathéter selon l'une des revendications précédentes, dans lequel :
le segment de succion (3.1.2) s'élargit de forme conique vers l'extrémité proximale du boîtier (3.1), et
le segment de sortie (3.1.4) se rétrécit de forme conique vers l'extrémité proximale du boîtier (3.1).

10. Dispositif de cathéter selon l'une des revendications 1 ou 2, comportant
un segment de connexion distal (3.1.1) entre l'extrémité distale (2) du boîtier et le segment de succion (3.1.2) ;
un segment de connexion proximal (3.1.5) entre le segment de sortie (3.1.4) et l'extrémité proximale du boîtier ;
le segment de connexion distal et le segment de connexion proximal (3.1.1, 3.1.5) présentent des ouvertures (3.1.7.2) qui sont plus grandes que les ouvertures (3.1.7.1) dans le segment de pompe (3.1.3).

11. Dispositif de cathéter selon l'une des revendications précédentes, dans lequel le rotor (3.2) est extensible et compressible de sorte que le rotor présente, à l'état comprimé, un diamètre qui correspond environ au diamètre intérieur du boîtier de pompe (3.1) et le rotor est distant, à l'état expansé, environ d'une distance comprise entre 0,01 mm et 0,5 mm du diamètre intérieur du boîtier de pompe.

12. Dispositif de cathéter selon l'une des revendications précédentes, comportant un tuyau flexible (29) qui entoure la tête de pompe (3) à l'état comprimé.

13. Dispositif de cathéter selon la revendication 12, dans lequel le boîtier de pompe (3.1) et le rotor sont réalisés de sorte que qu'ils se déploient radialement vers l'extérieur après que le tuyau flexible (29) ait été retiré.

14. Dispositif de cathéter selon la revendication 12 ou 13, dans lequel le rotor (3.2) et le boîtier (3.1) peuvent, à l'état comprimé, être introduits dans le tuyau flexible (29).
